(19) Europäisches Patentamt
European Patent Office
Office européen des brevets

(11) Veröffentlichungsnummer : **0 071 564**
**B1**

(12) # EUROPÄISCHE PATENTSCHRIFT

(45) Veröffentlichungstag der Patentschrift : **26.03.86**

(51) Int. Cl.[4] : **A 61 K 49/00**, C 07 C101/26, C 07 F 9/38, G 01 N 24/02

(21) Anmeldenummer : **82730097.1**

(22) Anmeldetag : **19.07.82**

(54) **Paramagnetische Komplexsalze, deren Herstellung und Verwendung bei der NMR-Diagnostik.**

(30) Priorität : **24.07.81 DE 3129906**

(43) Veröffentlichungstag der Anmeldung : **09.02.83 Patentblatt 83/06**

(45) Bekanntmachung des Hinweises auf die Patenterteilung : **26.03.86 Patentblatt 86/13**

(84) Benannte Vertragsstaaten : **AT BE CH DE FR GB IT LI LU NL SE**

(56) Entgegenhaltungen :
**DE-A- 2 527 158**
**DE-A- 2 918 842**
**FR-A- 1 111 504**
**FR-A- 2 159 411**
**FR-M- 484**
**FR-M- 988**
**GB-A- 2 008 944**
**US-A- 4 066 743**
**US-A- 4 125 599**
**US-A- 4 167 564**
**JOURNAL OF THE AMERICAN CHEMICAL SOCIETY, Band 99, Nr. 6, 16. März 1977, Seiten 1762-1765, Washington D.C., USA G.A. ELGAVISH et al.: "Aqueous lanthanide shift reagents. 3. Interaction of the ethylenediaminetetraacetate chelates with substituted ammonium cations"**
**I. Am. Chem. Soc. 96, 1976, 3726-3728, nisb. 2736**
**I. Am. Chem. Soc. 96, 678-681 (1974)**
**Frontreis of Biological Energetics, Vol. I, 752-759**
**J. of Computer Assisted Tomography 5 (2), 295-296**
**Phil. Trans R. Soc. London B 289, 483-487 (1980)**
**Radiology 91, 1190-1203 (1968)**
**Rote Liste 1977/78, Kap. 12 Antidota**
**J. Chem. Soc. (A) 1968, 1384**
**Refered ans Zh. Khim 1967, part I Abstr. No. 13 V 75**
**I. Am. Chen. Soc. 1977, 99 (6), 1762-1765**

**Die Akte enthält technische Angaben die nach dem Eingang der Anmeldung eingereicht wurden und die nicht in dieser Patentschrift enthalten sind.**

(73) Patentinhaber : **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder : **Gries, Heinz, Dr.**
**Helmstedter Strasse 19**
**D-1000 Berlin 31 (DE)**
Erfinder : **Rosenberg, Douwe, Dr.**
**Pariser Strasse 9**
**D-1000 Berlin 15 (DE)**
Erfinder : **Weinmann, Hanns-Joachim, Dr.**
**Westhofener Weg 27**
**D-1000 Berlin 38 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 071 564 B1

**Beschreibung**

Die Erfindung betrifft neue physiologisch verträgliche paramagnetische Komplexsalze, ihre Verwendung als NMR-Diagnostika, diese Verbindungen enthaltende Mittel sowie Verfahren zur Herstellung dieser Mittel.

Diese Komplexsalze bestehen aus Aminopolycarbonsäuren der Formeln I bis IV

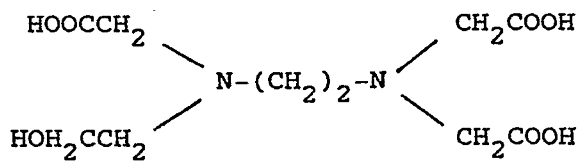

(I)

N-Hydroxyäthyl-N,N',N'-äthylendiamin-triessigsäure (HEDTA),

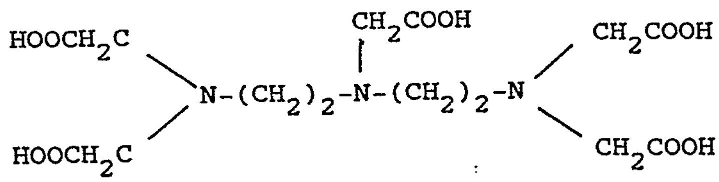

(II)

N,N,N',N'',N''-Diäthylentriamin-pentaessigsäure (DTPA),

$$HOH_2C—CH_2N(CH_2COOH)_2 \qquad (III)$$

N-Hydroxyäthyliminodiessigsäure,

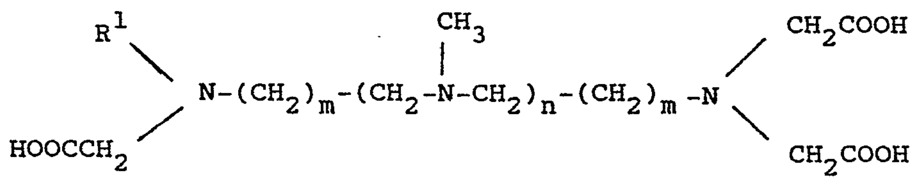

(IV)

worin

m die Zahlen 1 bis 4,

n die Zahlen 0 bis 2,

$R^1$ einen gesättigten oder ungesättigten Kohlenwasserstoffrest mit 4 bis 12 Kohlenwasserstoffatomen oder die Gruppe $—CH_2—COOH$ darstellt,

oder Diphosphonsäuren der allgemeinen Formel V

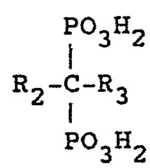

(V)

worin

$R_2$ Wasserstoff, Alkyl mit 1 bis 4 Kohlenstoffatomen, Halogen, die Hydroxy-, Amino- oder $CH_2—COOH$-Gruppe und

$R_3$ Wasserstoff, Alkyl mit 1 bis 4 Kohlenstoffatomen oder die $—CH_2—COOH$-Gruppe bedeuten,

und den Ionen der Lanthaniden-Elemente der Ordnungszahlen 57 bis 70 oder den Ionen der Übergangsmetalle der Ordnungszahlen 21 bis 29, 42 und 44

und einer organischen Base, wobei als organische Base Glucamin, N-Methylglucamin, N,N-Dimethylglucamin, Äthanolamin, Diäthanolamin, Morpholin, Lysin, Ornithin und Arginin in Betracht kommen.

Bevorzugte Komplexbildner sind N,N,N',N'-Äthylendiamin-tetraessigsäure (EDTA), N,N,N',N'',N''-Diäthylentriamin-pentaessigsäure (DTPA) sowie Äthan-1-hydroxy-1,1-diphosphonsäure, Methan-diphosphonsäure und Äthan-1-amino-1,1-diphosphonsäure.

Die bisher bekannten Komplexsalze enthalten weder die oben genannten Basen noch sind sie als NMR-Diagnostika verwendet worden. So sind zum Beispiel Praseodym-, Neodym-, Europium-EDTA- bzw. Praseodym-, Gadolinium-, Ytterbium-EDTA-Ammonium- und Pyridinium-Komplexe, wie in J. Am. Chem. Soc. *98*, 3726 (1976) bzw. in J. Am. Chem. Soc. *99*, 1762 (1977) beschrieben, nur für physikalische Untersuchungen eingesetzt worden.

Anwendung in der Humanmedizin fanden bisher nur die Kupfer-, Kobalt- und Eisen-Metallsalze der EDTA : In der Patentschrift FR-A-988 M (Laboratoires Gerda) werden das Kupfer-(II)-EDTA und dessen

Dinatriumsalz als Mittel zur Behandlung des Rheumatismus beschrieben ; die Patentschrift FR-A-484 M (Fabriques de produits chimiques Billault) befaßt sich mit dem Eisen(II)-Salz des Eisen(III)-Komplexes der EDTA als Mittel gegen Anämie ; in der Patentschrift FR-A-1 111 504 (Cassella Farbwerke Mainkur) werden das Dinatrium- und Dikaliumsalz des Kobalt-Komplexes der EDTA als Therapeutikum zur Blutbildung beschrieben. Für die NMR-Diagnostik sind diese Komplexsalze nicht verwendet worden.

Wenn erforderlich, ist es auch möglich, die erfindungsgemäßen Komplexverbindungen an Biomoleküle zu binden, um so die Komplexverbindungen an bestimmte Stellen des lebenden Körpers transportieren zu lassen.

Als Biomoleküle werden beispielsweise verwendet Immunglobuline, Hormone wie Insulin, Glucagen, Prostaglandine, Steroidhormone, Proteine, Peptide, Aminozucker, Lipide.

Die Kopplung der paramagnetischen Komplexsalze an die gewünschten Biomoleküle erfolgt nach an sich bekannten Methoden beispielsweise durch Reaktion der nucleophilen Gruppe eines Biomoleküles wie der Amino-, Phenol-, Sulfhydryl- oder Imidazolgruppe mit einem aktivierten Derivat der Komplexverbindung.

Als aktiviertes Derivat kommen beispielsweise Säurechloride, gemischte Anhydride (herstellbar aus dem Carboxylderivat der Komplexverbindung mit Chlorkohlensäureester), aktivierte Ester, Nitrene oder Isothiocyanate infrage.

Ebenso ist es möglich, ein aktiviertes Derivat des Biomoleküles mit einem nucleophilen Derivat der Komplexverbindung zur Reaktion zu bringen.

Als organische Basen werden zur Salzbildung verwendet : Glucamin, N-Methylglucamin, N,N-Dimethylglucamin, Äthanolamin, Diäthanolamin, Morpholin, wobei N-Methylglucamin bevorzugt ist. Zur Salzbildung geeignet sind auch basische Aminosäuren, wie zum Beispiel Lysin, Ornithin, Arginin.

Die Herstellung der neuen Mittel erfolgt in an sich bekannter Weise, indem man das paramagnetische Komplexsalz in Wasser oder physiologischer Salzlösung löst, gegebenenfalls unter Zusatz von in der Galenik üblichen Zusätzen wie beispielsweise physiologisch verträglichen Pufferlösungen (z. B. Natriumdihydrogenphosphatlösung) und die Lösung sterilisiert. Die wäßrigen Lösungen können oral, neural und insbesondere intravasal appliziert werden. Sind besonders für die orale Verabreichung Suspensionen der paramagnetischen Komplexsalze in Wasser oder physiologischer Salzlösung erwünscht, wird das paramagnetische Komplexsalz mit einem oder mehreren in der Galenik üblichen Hilfsstoffen und/oder Tensiden und/oder Aromastoffen zur Geschmackskorrektur gemischt und vor der oralen Anwendung in Wasser oder physiologischer Salzlösung suspendiert. Man verwendet hierbei vorzugsweise 3 bis 10 g paramagnetisches Komplexsalz und 2 bis 8 g eines oder mehrerer Hilfsstoffe wie beispielsweise Saccharose, hochdisperses Siliziumdioxid, Polyoxyäthylenpolyoxypropylen-Polymere, Stärke, Magnesiumstearat, Natriumlaurylsulfat, Talkum, Lactose, Carboxymethylcellulose-Natrium.

Zur NMR-Diagnostik beim Menschen werden wäßrige Lösungen oder Suspensionen verwendet, die 5 bis 250 mMol/l, vorzugsweise 50 bis 200 mMol/1, eines paramagnetischen Komplexsalzes enthalten. Die wäßrigen Lösungen liegen im pH-Bereich zwischen etwa 6,5 und 8,0, vorzugsweise zwischen 6,5 und 7,5.

Durch die Komplexbildung gemäß vorliegender Erfindung werden die paramagnetischen Salze entgiftet, und es wird außerdem erreicht, daß die Salze auch im physiologischen pH-Bereich in Wasser beständig und gut löslich sind.

Besonders geeignet erscheinen die neuen Mittel in Form der Komplexsalzlösungen zur besseren Abgrenzung bzw. Lokalisation von Läsionen der Bauchspeicheldrüse und der Leber sowie von Tumoren und Blutungen im cranialen Bereich. Zur Diagnose des zu untersuchenden Areals wird beispielsweise eine wäßrige, zum Blut isotonische Lösung paramagnetischer Komplexsalze in einer Dosis von 1 bis 100 µMol/kg intravenös appliziert. Bei einer Konzentration des Komplexsalzes von 50 bis 200 mMol/l werden für eine Untersuchung am Menschen etwa 1 bis 50 ml Lösung benötigt. Die Aufnahme der interessierenden Schicht erfolgt etwa 15 bis 60 Minuten nach der intravenösen Applikation der wäßrigen Lösung des paramagnetischen Komplexsalzes.

Die in der medizinischen Praxis üblichen physikalischen Diagnoseverfahren, die ohne oder nur mit geringfügigen operativen Eingriffen ausgeführt werden können, sind z. B. die Durchstrahlung des Körpers mit Röntgenlicht, die Szintigraphie und die Sonographie. Alle diese Methoden sind entweder mit gesundheitlichen Risiken behaftet oder der Anwendungsbereich ist eingeschränkt. So ist der Patient bei den Röntgentechniken und bei der Szintigraphie der ionisierenden Strahlung ausgesetzt, so daß diese Methoden nicht beliebig oft oder gar nicht bei Risikogruppen, wie beispielsweise bei Säuglingen und Schwangeren angewendet werden können.

Die Sonographie besitzt zwar die genannten Nachteile nicht, dafür ist aber ihr Anwendungsbereich sehr beschränkt, insbesondere im cranialen Bereich.

Da es trotz großem Forschungsaufwand bisher noch nicht gelungen ist, die geschilderten Nachteile vollständig zu beseitigen, sucht man nach bildgebenden Verfahren, die diese Nachteile nicht besitzen, aber einen vergleichbaren Informationsgewinn für die Diagnose liefern.

Eines dieser bildgebenden Verfahren ist die Kernspin-Tomographie (Spin-Imaging, Zeugmatographie), die auf dem physikalischen Effekt der sogenannten Kernspinresonanz (Nuclear Magnetic Resonance, NMR) beruht. Dieses Diagnoseverfahren ermöglicht es, Schnittbilder des lebenden Körpers und Einblick in Stoffwechselvorgänge zu erhalten ohne Anwendung ionisierender Strahlen. Den Effekt der Kernresonanz zeigen Atomkerne, die — wie Wasserstoff, der im biologischen Gewebe hauptsächlich

als Wasser vorhanden ist — ein magnetisches Moment besitzen und sich dadurch in einem starken äußeren Magnetfeld ausrichten. Durch einen Hochfrequenz-Impuls (Resonanzfrequenz) werden sie aus ihrer Gleichgewichtslage gebracht, in die sie mit einer charakteristischen Geschwindigkeit wieder zurückkehren. Die Dauer der Rückkehr in den Gleichgewichtszustand, die sogenannte Relaxationszeit, gibt Auskunft über den Ordnungsgrad der Atome und über ihre Wechselwirkung mit ihrer Umgebung.

Die bildliche Darstellung, die man durch die Messung der Protonendichte bzw. der Relaxationszeiten erhält, ist von hohem diagnostischen Wert und gibt Auskunft über den Wassergehalt und über den Zustand des untersuchten Gewebes. So zeigt Tumorgewebe beispielsweise längere Relaxationszeiten als gesundes Vergleichsgewebe. (A. Ganssen u.a. Computertomographie 1, [1981], S. 2-10 ; Georg Thieme Verlag, Stuttgart, New York).

Man hat nun festgestellt, daß paramagnetische Ionen, wie beispielsweise $Mn^{2}+$ (Mangan) oder $Cu^{2}+$ (Kupfer) die Relaxationszeiten beeinflussen und damit den Informationsgehalt erhöhen.

Die bisher hierfür am Versuchstier verwendeten Schwermetallsalzlösungen sind jedoch für die intravenöse Applikation am Menschen wegen ihrer hohen Toxizität ungeeignet. Man sucht daher nach paramagnetischen Substanzen, die gut vertragen werden und die Bildgebung günstig beeinflussen. Letzteres kann beispielsweise dadurch erfolgen, daß möglichst organspezifisch die Spin-Gitter-Relaxationszeit $T_1$ stark herabgesetzt wird, während gleichzeitig die Spin-Spin-Relaxationszeit $T_2$ weitgehend konstant gehalten wird. Es wurde nun gefunden, daß die gewünschte Entgiftung der sonst toxischen Metallsalze durch eine Komplexierung erfolgen kann, ohne daß die paramagnetischen Eigenschaften ungünstig beeinflußt werden. Letzteres ist überraschend, da hierdurch bekanntlich die Verteilung der d- bzw. f-Elektronen über die d- bzw. f-Orbitale verändert wird.

So konnte bei Versuchen an Ratten im Scanner mit einem Magnetfeld von 0,15 Tesla bei einer eingespeisten Energie von 300 Watt/Impuls und einem 180°-Puls von 720 µs bei Aufnahmezeiten von 2 Minuten jeweils 10 Minuten nach intravenöser Injektion von 20 µMol/kg Mangan-Edetat als wäßrige Methylglucaminsalzlösung einer Konzentration von 6 mMol/l gegenüber der Leeraufnahme eine deutlich stärkere Änderung des Signals im Bereich des Leberparenchyms beobachtet werden, während mit einer wäßrigen Mangan(II)-chloridlösung gleicher Molarität unter denselben Versuchsbedingungen nur eine vergleichsweise geringe Kontrastierung erzielt werden konnte. Andererseits erreicht man durch die Komplexbildung die gewünschte Entgiftung der sonst toxischen paramagnetischen Salze. So wurde bei der Ratte nach intravenöser Injektion einer wäßrigen Lösung des N-Methylglucaminsalzes von Mangan-Edetat eine $LD_{50}$ von 4 mMol/kg ermittelt. Demgegenüber zeigte Manganchlorid unter identischen Bedingungen an der Ratte eine $LD_{50}$ von nur 0,5 mMol/kg.

Die Durchführung einer NMR-diagnostischen Untersuchung unter Verwendung eines Komplexsalzes wird anhand des folgenden Beispiels näher erläutert :

Es wird eine sterile, wäßrige Lösung des N-Methylglucaminsalzes des Gadolinium-III-Komplexes der Diäthylentriaminpentaessigsäure mit einer Konzentration von 0,1 Mol/l hergestellt. Der pH-Wert der klaren Lösung beträgt 7,2.

Der für die NMR-Tomographie benutzte (Siemens AG/Erlangen) Ganzkörpercanner arbeitet mit einem Magnetfeld von 0,1 T, das einer Larmorprotonenfrequenz von 4,99 MHz entspricht. Das Gerät wurde mit einer Hochfrequenzsende- und empfangsspule kleinerer Abmessung ausgestattet, um auch Objekte geringerer Größe mit befriedigender Auflösung abbilden zu können. Die Untersuchungen werden nach einem Spin-Echo-Verfahren durchgeführt. Die Zeit einer Aufnahme beträgt zwischen 1 und 3 Minuten.

Die Experimente werden mit männlichen Ratten der Zucht Wistar-Han-Shering (SPF) mit einem Körpergewicht von 250 g durchgeführt. Acht Tage vor der Untersuchung wird den Tieren eine Novikoff-Hepatoma-Tumor-Zellsuspension intraperitoneal verabreicht (0,5 ml mit $1 \times 10^6$ Zellen).

Die Tiere werden durch eine intraperitoneale Injektion von Pentobarbital-Natrium narkotisiert (60 mg/kg Körpergewicht). Anschließend wir den Tieren eine Flügelkanüle in eine der Schwanzvenen gelegt.

Vor der Applikation des Kontrastmittels werden Aufnahmen in sagittalen und horizontalen Ebenen des Körperstammes angefertigt.

Das Kontrastmittel wird innerhalb von einer Minute in einer Dosis von 1 mMol/kg intravenös appliziert ; etwa 22-25 min p. appl., ist eine starke Helligkeitserhöhung im Abdomen festzustellen. Nach intravenöser Gabe gelangt das Kontrastmittel in die pathologischen Flüssigkeitsansammlungen und bewirkt hier eine starke Verkürzung der Spin-Gitter-Relaxationszeit ($T_1$), die zur Steigerung der Signalintensität führt. Erst nach Applikation des Kontrastmittels ist die tumoröse Flüssigkeitsansammlung und eine verbesserte Abgrenzung der Organe erkennbar. Ohne Kontrastmittelgabe sind kaum Strukturen im Abdomen zu erkennen, da die Organe nur geringe Unterschiede in Protonendichte und Relaxationszeiten zeigen.

Auch nach oraler Gabe des Kontrastmittels wird die Abgrenzung von Strukturen verbessert. Hierzu werden 5 ml der N-Methylglucaminlösung des Gadoliniumkomplexes der Diäthylentriaminpentaessigsäure in einer Konzentration von 1 mMol/l mit Hilfe einer Sonde einer narkotisierten männlichen Ratte (Körpergewicht : 250 g) verabreicht. Erst nach Gabe des Kontrastmittels ist eine klare Abgrenzung des Magens bzw. des Darmtraktes von den restlichen Organen sichtbar.

Eigene pharmakokinetische Untersuchungen an der Ratte haben gezeigt, daß das N-Methylglucamin

des Gadoliniumkomplexes der Diäthylentriaminpentaessigsäure nach intravenöser und subkanter Gabe innerhalb von 24 h ganz überwiegend renal eliminiert wird. Der Gadoliniumkomplex wird durch glomeruläre Filtration mit einer Halbwertzeit von etwa 20 min von der Ratte ausgeschieden. Der mit den Faeces eliminierte Anteil ist kleiner als 5 % der applizierten Dosis.

Nach oraler Gabe wird keine Resorption der Substanz beobachtet. Das pharmakokinetische Verhalten ähnelt dem der klassischen Röntgenkontrastmittel für die Uroangiographie.

Die Herstellung der paramagnetischen Komplexsalze erfolgt nach dem Fachmann bekannten Verfahren, indem man das paramagnetische Metallsalz der Lanthaniden-Elemente der Ordnungszahlen 57 bis 70 oder der Übergangsmetalle der Ordnungszahlen 21 bis 29, 42 und 44 in Wasser und/oder Alkohol löst und mit der Lösung der äquivalenten Menge des Komplexbildners in Wasser und/oder Alkohol versetzt und rührt, falls erforderlich unter Erhitzen auf 50 °C bis 120 °C bis die Umsetzung beendet ist. Wird Alkohol als Lösungsmittel verwendet, so wird Methanol oder Äthanol benutzt.

Wenn der gebildete Komplex im verwendeten Lösungsmittel unlöslich ist, kristallisiert er und kann abfiltriert werden. Ist er löslich, kann er durch Eindampfen der Lösung zur Trockne isoliert werden.

Das Verfahren soll anhand der folgenden Arbeitsvorschriften näher erläutert werden :

Herstellung des Mangan-II-Komplexes der Äthylendiamintetraessigsäure :

Die Suspension von 6,17 g Mangan-II-carbonat in 500 ml Wasser wird mit 14,6 g Äthylendiamintetraessigsäure versetzt und unter Rühren auf dem Dampfbad erwärmt, wobei eine Gasentwicklung auftritt. Die anfänglich rosa Farbe verschwindet nach ca. 20 Minuten und es geht alles bis auf einen kleinen Rest in Lösung. Nach einer Stunde Rühren bei 110 °C wird vom Ungelösten abfiltriert und das Filtrat abgekühlt. Nach 15 Stunden Stehen wird das Kristallisat abgesaugt und getrocknet :

$K_1$ = 14,1 g (Molgewicht 345,17)

Fp. : 256°/258-259 °C.

Herstellung des Gadolinium-III-Komplexes der Diäthylentriamin-pentaessigsäure :

Die Suspension von 435 g Gadoliniumoxid ($Gd_2O_3$) und 944 g Diäthylentriamin-pentaessigsäure in 12 Liter Wasser wird unter Rühren auf 90 °C bis 100 °C erwärmt und 48 Stunden bei dieser Temperatur gerührt. Dann filtriert man vom Ungelösten ab und dampft das Filtrat zur Trockne.

Der amorphe Rückstand wird pulverisiert.

Ausbeute 144 g ; (Molargewicht 547,58)

Fp. : schmilzt ab 235° und bleibt bis 320 °C unzersetzt.

Ist (Sind) in der erhaltenen paramagnetischen Komplexverbindung noch eine oder mehrere saure Gruppe(n) enthalten, so kann die erhaltene Komplexverbindung anschließend in Wasser gelöst oder suspendiert und mit der gewünschten organischen Base versetzt werden, bis der Neutralpunkt erreicht ist. Nach Filtration von ungelösten Anteilen wird die Lösung eingedampft und als Rückstand das gewünschte Komplexsalz erhalten.

Die Erfindung betrifft die paramagnetischen Komplexsalze gemäß den Ansprüchen 1 bis 9, ihre Verwendung als NMR-Diagnostika, diese Verbindungen enthaltende Mittel sowie Verfahren zur Herstellung dieser Mittel.

Die nachfolgenden Beispiele sollen die Erfindung näher erläutern.

## Beispiel 1

Herstellung vom Di-N-methylglucaminsalz des Mangan(II)-Komplexes der Äthylendiamin-tetraessigsäure, $C_{24}H_{48}N_4O_{18}Mn$

7,4 g (= 20 mMol) des Mangan(II)-Komplexes der Äthylendiamin-tetraessigsäure (Wassergehalt : 6,9 %) werden in 30 ml Wasser suspendiert und durch Zugabe von rund 7,8 g (= rund 40 mMol) N-Methylglucamin bei pH 7,5 gelöst. Nach Filtration von wenig Ungelöstem wird die Lösung im Vakuum zur Trockne eingeengt. In quantitativer Ausbeute entsteht ein fester Schaum, der ab 95 °C schmilzt und bei 170 °C zähflüssig wird.

Analyse in der Trockensubstantz :

ber. C 39,19 % H 6,58 % N 7,61 % Mn 7,47 %

gef. C 39,23 % H 7,10 % N 7,26 % Mn 7,53 % $H_2O$ 3,26 %

Äquivalentgewicht ber. 367,8 gef. 369 (Titration mit Tetramethylammoniumhydroxid in wäßrigem Aceton).

Durch Lösen in heißem Äthanol und Einengen im Vakuum zur Trockne erhält man die Substanz als weißes, hygroskopisches Pulver.

In analoger Weise werden erhalten :

Di-N-methylglucaminsalz des Nickel(II)-Komplexes der Äthylendiamin-tetraessigsäure, $C_{24}H_{48}N_4O_{18}Ni$ als blaues Pulver.

Di-äthanolaminsalz des Kobalt(II)-Komplexes der Äthylendiamin-tetraessigsäure, $C_{14}H_{28}N_4O_{10}Co$ als rosafarbenes Pulver.

Di-morpholinsalz des Mangan(II)-Komplexes der Äthylendiamin-tetraessigsäure, $C_{18}H_{32}N_4O_{10}Mn$ als weißes Pulver.

Di-diäthanolaminsalz des Kupfer(II)-Komplexes der Äthylendiamin-tetraessigsäure, $C_{18}H_{36}N_4O_{12}Cu$ als blaues Pulver.

Tri-diäthanolaminsalz des Mangan(II)-Komplexes der Diäthylentriaminpentaessigsäure, $C_{26}H_{54}N_6O_{16}Mn$ als gelbes Pulver.

Tri-N-methylglucaminsalz des Mangan(II)-Komplexes der Diäthylentriaminpentaessigsäure, $C_{35}H_{72}N_6O_{25}Mn$ als weißes Pulver.

Beispiel 2

Herstellung vom N-Methylglucaminsalz des Gadolinium(III)-Komplexes der Äthylendiamin-tetraessigsäure, $C_{17}H_{30}N_3O_{13}Gd$

4,58 g (= 10 mMol) des Gadolinium(III)-Komplexes der Äthylendiamin-tetraessigsäure (Wassergehalt : 2,7 %) werden in 15 ml Wasser suspendiert und durch Zugabe von 1,95 g (= 10 mMol) N-Methylglucamin bei pH 7,4 gelöst. Die Lösung wird filtriert und anschließend im Vakuum zur Trockne eingeengt, wobei ein fester Schaum entsteht. Die Ausbeute ist unter Berücksichtigung des Wassergehaltes von 8,5 % praktisch quantitativ. Die Substanz sintert ab 90 °C, ab 140 °C tritt Schaumentwicklung ein.

Analyse in der Trockensubstanz :

ber. C 26,90 % H 2,44 % N 6,27 % Gd 35,22 %

gef. C 26,78 % H 2,96 % N 5,77 % Gd 34,99 %

Äquivalentgewicht ber. 641,7, gef. 634 (Titration mit Tetramethylammoniumhydroxid in wäßrigem Aceton).

Durch Lösen in heißem Äthanol und Einengen im Vakuum zur Trockne erhält man die Substanz als weißes Pulver.

In analoger Weise werden erhalten :

N-Methylglucaminsalz des Dysprosium(III)-Komplexes der Äthylendiamintetraessigsäure, $C_{17}H_{30}N_3O_{13}Dy$ ;

Di-N-methylglucaminsalz des Holmium(III)-Komplexes der Diäthylentriamin-pentaessigsäure, $C_{28}H_{54}N_5O_{20}Ho$ ;

Di-Lysinsalz des Gadolinium-III-Komplexes der Diäthylentriaminpentaessigsäure, $C_{26}H_{48}N_7O_{14}Gd$ ;

N-Methyl-glucaminsalz des Gadolinium-III-Komplexes der Diäthylentriaminpentaessigsäure, $C_{28}H_{54}N_5O_{20}Gd$.

Beispiel 3

Herstellung einer Lösung vom Di-N-methylglucaminsalz des Mangan(II)-Komplexes der Äthylendiamintetraessigsäure

3,68 g (= 5 mMol) der in Beispiel 1 beschriebenen Substanz werden in 70 ml Wasser pro injectione (p.i.) gelöst und die Lösung mit 0,4 g Natriumchlorid versetzt. Anschließend wird auf 100 ml mit Wasser p.i. aufgefüllt und die Lösung durch ein Sterilfilter in Ampullen abgefüllt. Die Lösung ist mit 280 mOsm. blutisoton.

Beispiel 4

Herstellung einer Lösung vom N-Methylglucaminsalz des Gadolinium(III)-Komplexes der Äthylendiamintetraessigsäure

9,63 g (= 15 mMol) der in Beispiel 2 beschriebenen Substanz werden in 100 ml Wasser p.i. gelöst. Die annähernd blutisotone Lösung wird über ein Sterilfilter in Ampullen abgefüllt.

Beispiel 5

Herstellung einer Lösung vom Di-N-methylglucaminsalz des Gadolinium(III)-Komplexes der Diäthylentriaminpentaessigsäure

5,35 g (= 9 mMol) des Gadolinium(III)-Komplexes der Diäthylentriaminpentaessigsäure (Wassergehalt 8 %) werden in 50 ml Wasser p.i. gelöst und durch Zusatz von rund 3,2 g (entsprechend rund 18 mMol) N-Methylglucamin bis pH 7,5 neutralisiert. Anschließend wird die Lösung mit Wasser p.i. auf 100 ml aufgefüllt, in Ampullen abgefüllt und hitzesterilisiert. Die Konzentration der Lösung ist auf Blutisotonie (rund 280 mOsm.) eingestellt.

## Beispiel 6

Herstellung einer Lösung vom Di-N-methylglucaminsalz des Dysprosium(III)-Komplexes der Diäthylentriaminpentaessigsäure

8,00 g (= 15 mMol) des Dysprosium(III)-Komplexes der Diäthylentriaminpentaessigsäure werden in 80 ml Wasser p.i. unter Zusatz von rund 5,3 g (entsprechend rund 30 mMol) N-Methylglucamin bei pH 7,5 gelöst. Anschließend wird die Lösung mit Wasser p.i. auf 170 ml aufgefüllt. Die annähernd blutisotone Lösung wird ampulliert und hitzesterilisiert.

## Beispiel 7

Herstellung einer Lösung vom Di-N-methylglucaminsalz des Holmium(III)-Komplexes der Diäthylentriamin-pentaessigsäure

8,02 g (= 15 mMol) des Holmium(III)-Komplexes der Diäthylentriaminpentaessigsäure werden in 80 ml Wasser p.i. unter Zusatz von rund 5,3 g (entsprechend rund 30 mMol) N-Methylglucamin bei pH 7,2 gelöst. Anschließend wird die Lösung mit Wasser p.i. auf 170 ml aufgefüllt. Die annähernd blutisotone Lösung wird ampulliert und hitzesterilisiert.

Die Lösung kann auch durch Auflösen des gemäß Beispiel 2 isolierten Komplexsalzes in Wasser p.i. hergestellt werden.

## Beispiel 8

Herstellung einer Lösung vom N-Methylglucaminsalz des Eisen(II)-Komplexes der Äthan-1-hydroxy-1,1-diphosphonsäure

1,27 g (= 10 mMol) Eisen(II)-chlorid werden in 8,8 ml Methanol gelöst und die Lösung mit 3,2 ml einer 60 gew.-%igen Lösung von Äthan-1-hydroxy-1,1-diphosphonsäure in Wasser versetzt. Man engt die Lösung im Vakuum zur Trockne ein und wäscht den Rückstand dreimal mit wasserfreiem Methanol. Nach dem Trocknen wird der Rückstand in 50 ml Wasser p.i. aufgenommen und durch Zusatz von rund 1,8 g (entsprechend rund 10 mMol) N-Methylglucamin bei pH 7,5 gelöst. Anschließend wird die Lösung mit Wasser p.i. auf 100 ml aufgefüllt und nach Sterilfiltration in Ampullen abgefüllt.

## Beispiel 9

Herstellung einer Lösung des Di-N-Methylglucaminsalzes des Mangan(II)-Komplexes der Hexandiyliminotetraessigsäure

Analog der in Beispiel 6 beschriebenen Arbeitsweise wird aus 6,02 g (~ 15 mMol) Mangan(II)-Komplex von Hexandiyldiamintetraessigsäure und 5,86 g (~ 30 mMol) N-Methylglucamin eine gebrauchsfertige Lösung hergestellt.

## Beispiel 10

(Zusammensetzung eines Pulvers zur Herstellung einer Suspension)

4,000 g Gadolinium-III-Komplex der Diäthylentriaminpentaessigsäure (Wassergehalt 8 %)
3,895 g Saccharose
0,100 g Polyoxyäthylenpolyoxypropylen-Polymeres
0,005 g Aromastoffe
8,000 g

**Patentansprüche** (für die Vertragsstaaten : BE, CH, DE, FR, GB, IT, LI, LU, NL, SE)

1. Physiologisch verträgliche paramagnetische Komplexsalze aus Aminopolycarbonsäuren der Formeln I bis IV

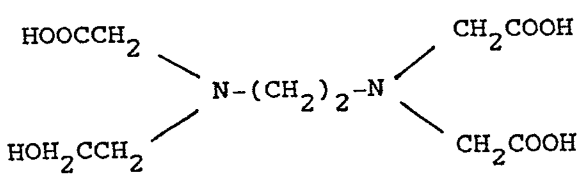

(I)

N-Hydroxyäthyl-N,N',N'-äthylendiamin-triessigsäure (HEDTA),

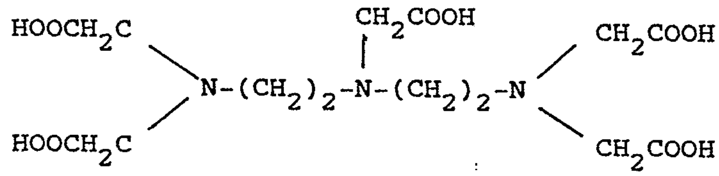

(II)

N,N,N',N'',N''-Diäthylentriamin-pentaessigsäure (DTPA),

$$HOH_2C\text{-}CH_2N(CH_2COOH)_2 \qquad \text{(III)}$$

N-Hydroxyäthyliminodiessigsäure,

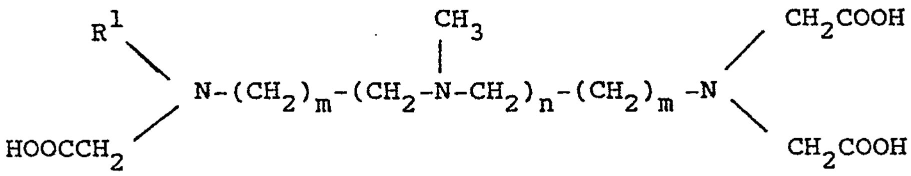

(IV)

worin

m die Zahlen 1 bis 4,

n die Zahlen 0 bis 2,

$R^1$ einen gesättigten oder ungesättigten Kohlenwasserstoffrest mit 4 bis 12 Kohlenwasserstoffatomen oder die Gruppe $—CH_2—COOH$ darstellt,

oder Diphosphonsäuren der allgemeinen Formel V

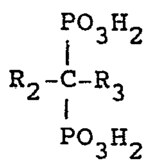

(V)

worin

$R_2$ Wasserstoff, Alkyl mit 1 bis 4 Kohlenstoffatomen, Halogen, die Hydroxy-, Amino- oder $CH_2—COOH—$Gruppe und

$R_3$ Wasserstoff, Alkyl mit 1 bis 4 Kohlenstoffatomen oder die $—CH_2—COOH—$Gruppe bedeuten, und den Ionen der Lanthaniden-Elemente der Ordnungszahlen 57 bis 70 oder den Ionen der Übergangsmetalle der Ordnungszahlen 21 bis 29, 42 und 44

und einer organischen Base, wobei als organische Base Glucamin, N-Methylglucamin, N,N-Dimethylglucamin, Äthanolamin, Diäthanolamin, Morpholin, Lysin, Ornithin und Arginin in Betracht kommen.

2. Physiologisch verträgliche paramagnetische Komplexsalze nach Anspruch 1, wobei als Aminopolycarbonsäure N,N,N',N'-Äthylendiamin-tetraessigsäure (EDTA) verwendet wird.

3. Physiologisch verträgliche paramagnetische Komplexsalze nach Anspruch 1, wobei als Aminopolycarbonsäure N,N,N',N'',N''-Diäthylentriaminpentaessigsäure (DTPA) verwendet wird.

4. Physiologisch verträgliche paramagnetische Komplexsalze nach Anspruch 1, wobei als Diphosphonsäure Äthan-1-hydroxy-1,1-diphosphonsäure, Methan-diphosphonsäure oder Äthan-1-amino-1,1-diphosphonsäure verwendet wird.

5. Di-N-methylglucaminsalz des Mangan(II)-Komplexes der Äthylendiamin-tetraessigsäure, Di-N-methylglucaminsalz des Nickel(II)-Komplexes der Äthylendiamin-tetraessigsäure, Tri-N-methylglucaminsalz des Mangan(II)-Komplexes der Diäthylentriamin-pentaessigsäure, N-Methylglucaminsalz des Gadolinium(III)-Komplexes der Äthylendiamin-tetraessigsäure, N-Methylglucaminsalz des Dysprosium(III)-Komplexes der Äthylendiamin-tetraessigsäure, Di-N-Methylglucaminsalz des Holmium(III)-Komplexes der Diäthylentriamin-pentaessigsäure und N-Methylglucaminsalz des Eisen(II)-Komplexes der Athan-1-hydroxy-1,1-diphosphonsäure.

6. Di-N-Methylglucaminsalz des Gadolinium(III)-Komplexes der Diäthylentriamin-pentaessigsäure.

7. Di-äthanolaminsalz des Kobalt(II)-Komplexes der Äthylendiamin-tetraessigsäure, Di-diäthanolaminsalz des Kupfer(II)-Komplexes der Äthylendiamin-tetraessigsäure und Tri-diäthanolaminsalz des Mangan(II)-Komplexes der Diäthylentriamin-pentaessigsäure.

8. Di-morpholinsalz des Mangan(II)-Komplexes der Äthylendiamin-tetraessigsäure.

9. Di-Lysinsalz des Gadolinium(III)-Komplexes der Diäthylentriamin-pentaessigsäure.

10. Physiologisch verträgliche paramagnetische Komplexsalze aus Aminopolycarbonsäuren der Formeln I bis IV

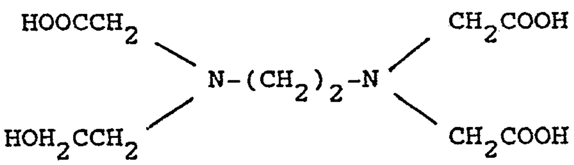

(I)

N-Hydroxyäthyl-N,N',N'-äthylendiamin-triessigsäure (HEDTA),

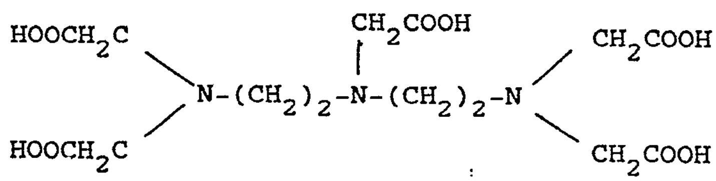

(II)

N,N,N',N'',N''-Diäthylentriamin-pentaessigsäure (DTPA),

$$HOH_2C—CH_2N(CH_2COOH)_2 \qquad (III)$$

N-Hydroxyäthyliminodiessigsäure,

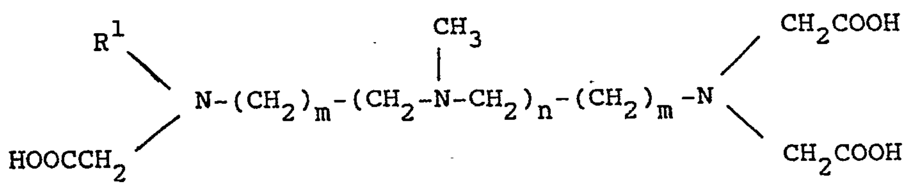

(IV)

worin

m die Zahlen 1 bis 4,

n die Zahlen 0 bis 2,

$R^1$ einen gesättigten oder ungesättigten Kohlenwasserstoffrest mit 4 bis 12 Kohlenwasserstoffatomen oder die Gruppe $—CH_2—COOH$ darstellt,

oder Diphosphonsäuren der allgemeinen Formel V

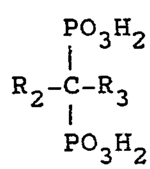

(V)

worin

$R_2$ Wasserstoff, Alkyl mit 1 bis 4 Kohlenstoffatomen, Halogen, die Hydroxy-, Amino- oder $CH_2—COOH$—Gruppe und

$R_3$ Wasserstoff, Alkyl mit 1 bis 4 Kohlenstoffatomen oder die $—CH_2—COOH$—Gruppe bedeuten,

und den Ionen der Lanthaniden-Elemente der Ordnungszahlen 57 bis 70 oder den Ionen der Übergangsmetalle der Ordnungszahlen 21 bis 29, 42 und 44

und einer organischen Base, wobei als organische Base Glucamin, N-Methylglucamin, N,N-Dimethylglucamin, Äthanolamin, Diäthanolamin, Morpholin, Lysin, Ornithin und Arginin in Betracht kommen, zur Anwendung in der NMR-Diagnostik.

11. Physiologisch verträgliche paramagnetische Komplexsalze nach Anspruch 10, wobei als Aminopolycarbonsäure N,N,N',N'-Äthylendiamin-tetraessigsäure (EDTA) verwendet wird, zur Anwendung in der NMR-Diagnostik.

12. Physiologisch verträgliche paramagnetische Komplexsalze nach Anspruch 10, wobei als Aminopolycarbonsäure N,N,N',N'',N''-Diäthylentriaminpentaessigsäure (DTPA) verwendet wird, zur Anwendung in der NMR-Diagnostik.

13. Physiologisch verträgliche paramagnetische Komplexsalze nach Anspruch 10, wobei als Diphosphonsäure Äthan-1-hydroxy-1,1-diphosphonsäure, Methan-diphosphonsäure oder Äthan-1-amino-1,1-diphosphonsäure verwendet wird, zur Anwendung in der NMR-Diagnostik.

14. Di-N-methylglucaminsalz des Mangan(II)-Komplexes der Äthylendiamin-tetraessigsäure, Di-N-methylglucaminsalz des Nickel(II)-Komplexes der Äthylendiamin-tetraessigsäure, Tri-N-methylglucaminsalz des Mangan(II)-Komplexes der Diäthylentriamin-pentaessigsäure, N-Methylglucaminsalz des Gadolinium(III)-Komplexes der Äthylendiamin-tetraessigsäure, N-Methylglucaminsalz des Dysprosium(III)-Komplexes der Äthylendiamin-tetraessigsäure, Di-N-Methylglucaminsalz des Holmium(III)-Komplexes

der Diäthylentriamin-pentaessigsäure und N-Methylglucaminsalz des Eisen(II)-Komplexes der Äthan-1-hydroxy-1,1-diphosphonsäure zur Anwendung in der NMR-Diagnostik.

15. Di-N-Methylglucaminsalz des Gadolinium(III)-Komplexes der Diäthylentriamin-pentaessigsäure zur Anwendung in der NMR-Diagnostik.

16. Di-äthanolaminsalz des Kobalt(II)-Komplexes der Äthylendiamin-tetraessigsäure, Di-diäthanolaminsalz des Kupfer(II)-Komplexes der Äthylendiamin-tetraessigsäure und Tri-diäthanolaminsalz des Mangan(II)-Komplexes der Diäthylentriamin-pentaessigsäure zur Anwendung in der NMR-Diagnostik.

17. Di-morpholinsalz des Mangan(II)-Komplexes der Äthylendiamin-tetraessigsäure zur Anwendung in der NMR-Diagnostik.

18. Di-Lysinsalz des Gadolinium(III)-Komplexes der Diäthylentriamin-pentaessigsäure zur Anwendung in der NMR-Diagnostik.

19. Mittel für die NMR-Diagnostik, enthaltend mindestens ein paramagnetisches, physiologisch verträgliches Komplexsalz aus Aminopolycarbonsäuren der Formeln I bis IV

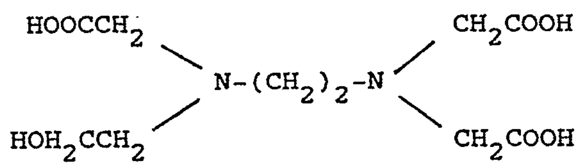

(I)

N-Hydroxyäthyl-N,N',N'-äthylendiamin-triessigsäure (HEDTA),

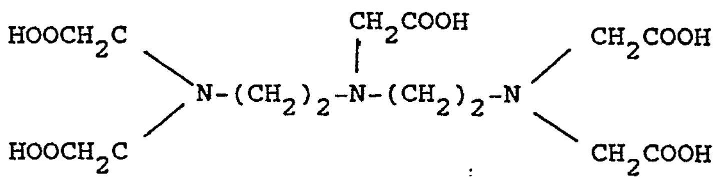

(II)

N,N,N',N'',N''-Diäthylentriamin-pentaessigsäure (DTPA),

$$HOH_2C—CH_2N(CH_2COOH)_2 \qquad (III)$$

N-Hydroxyäthyliminodiessigsäure,

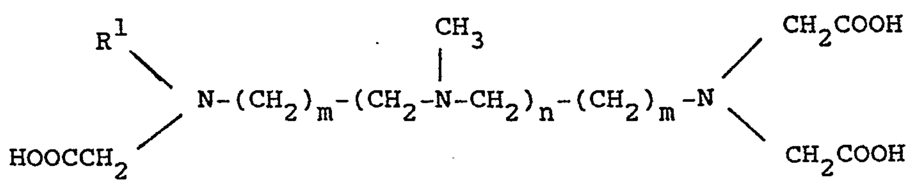

(IV)

worin

m die Zahlen 1 bis 4,

n die Zahlen 0 bis 2,

$R^1$ einen gesättigten oder ungesättigten Kohlenwasserstoffrest mit 4 bis 12 Kohlenwasserstoffatomen oder die Gruppe $—CH_2—COOH$ darstellt,

oder Diphosphonsäuren der allgemeinen Formel V

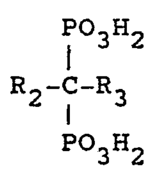

(V)

worin

$R_2$ Wasserstoff, Alkyl mit 1 bis 4 Kohlenstoffatomen, Halogen, die Hydroxy-, Amino- oder $CH_2—COOH$—Gruppe und

$R_3$ Wasserstoff, Alkyl mit 1 bis 4 Kohlenstoffatomen oder die $—CH_2—COOH$—Gruppe bedeuten,

und den Ionen der Lanthaniden-Elemente der Ordnungszahlen 57 bis 70 oder den Ionen der Übergangsmetalle der Ordnungszahlen 21 bis 29, 42 und 44

und einer organischen Base, wobei als organische Base Glucamin, N-Methylglucamin, N,N-Dimethylglucamin, Äthanolamin, Diäthanolamin, Morpholin, Lysin, Ornithin und Arginin in Betracht kommen, gegebenenfalls mit den in der Galenik üblichen Zusätzen, gelöst oder suspendiert in Wasser oder physiologischer Salzlösung.

20. Mittel für die NMR-Diagnostik nach Anspruch 19, wobei als Aminopolycarbonsäure N,N,N',N'-Äthylendiamin-tetraessigsäure (EDTA) verwendet wird.

21. Mittel für die NMR-Diagnostik nach Anspruch 19, wobei als Aminopolycarbonsäure N,N,N', N",N"-Diäthylentriamin-pentaessigsäure (DTPA) verwendet wird.

22. Mittel für die NMR-Diagnostik nach Anspruch 19, enthaltend mindestens ein paramagnetisches, physiologisch verträgliches Komplexsalz aus einer Aminopolycarbonsäure der Formel I bis IV und einem Ion der Lanthaniden-Elemente der Ordnungszahlen 57 bis 70 und einer organischen Base.

23. Mittel für die NMR-Diagnostik nach Anspruch 19, enthaltend mindestens ein paramagnetisches, physiologisch verträgliches Komplexsalz aus einer Aminopolycarbonsäure der Formel I bis IV und einem Ion der Übergangsmetalle der Ordnungszahlen 21 bis 29, 42 und 44 und einer organischen Base.

24. Mittel für die NMR-Diagnostik nach Anspruch 19, enthaltend mindestens ein paramagnetisches, physiologisch verträgliches Komplexsalz aus einer Diphosphonsäure der allgemeinen Formel V und einem Ion der Lanthaniden-Elemente der Ordnungszahlen 57 bis 70 und einer organischen Base.

25. Mittel für die NMR-Diagnostik nach Anspruch 19, enthaltend mindestens ein paramagnetisches, physiologisch verträgliches Komplexsalz aus einer Diphosphonsäure der allgemeinen Formel V und einem Ion der Übergangsmetalle der Ordnungszahlen 21 bis 29, 42 und 44 und einer organischen Base.

26. Mittel für die NMR-Diagnostik nach Anspruch 19, enthaltend mindestens ein physiologisch verträgliches paramagnetisches Komplexsalz, wobei als Diphosphonsäure Äthan-1-hydroxy-1,1-diphosphonsäure, Methan-diphosphonsäure oder Äthan-1-amino-1,1-diphosphonsäure verwendet wird.

27. Mittel für die NMR-Diagnostik nach Anspruch 19, enthaltend Di-N-methylglucaminsalz des Mangan (II)-Komplexes der Äthylendiamin-tetraessigsäure, Di-N-methylglucaminsalz des Nickel(II)-Komplexes der Äthylendiamin-tetraessigsäure, Tri-N-methylglucaminsalz des Mangan(II)-Komplexes der Diäthylentriaminpentaessigsäure, N-Methylglucaminsalz des Gadolinium-(III)-Komplexes der Äthylendiamin-tetraessigsäure, N-Methylglucaminsalz des Dysprosium(III)-Komplexes der Äthylendiamin-tetaessigsäure, Di-N-Methylglucaminsalz des Holmium(III)-Komplexes der Diäthylentriamin-pentaessigsäure oder N-Methylglucaminsalz des Eisen(II)-Komplexes der Äthan-1-hydroxy-1,1-diphosphonsäure.

28. Mittel für die NMR-Diagnostik nach Anspruch 19, enthaltend Di-N-Methylglucaminsalz des Gadolinium(III)-Komplexes der Diäthylentriamin-pentaessigsäure.

29. Mittel für die NMR-Diagnostik nach Anspruch 19, enthaltend Di-äthanolaminsalz des Kobalt(II)-Komplexes der Äthylendiamin-tetraessigsäure, Di-diäthanolaminsalz des Kupfer(II)-Komplexes der Äthylendiamin-tetraessigsäure oder Tri-diäthanolaminsalz des Mangan(II)-Komplexes der Diäthylentriaminpentaessigsäure.

30. Mittel für die NMR-Diagnostik nach Anspruch 19, enthaltend Di-morpholinsalz des Mangan(II)-Komplexes der Äthylendiamintetraessigsäure.

31. Mittel für die NMR-Diagnostik nach Anspruch 19, enthaltend Di-Lysinsalz des Gadolinium(III)-Komplexes der Diäthylentriamin-pentaessigsäure.

32. Mittel für die NMR-Diagnostik nach Anspruch 19 bis 31, dadurch gekennzeichnet, daß sie 5 bis 250 mMol/l Komplexsalz enthalten.

33. Verfahren zur Herstellung der Mittel gemäß Ansprüche 19 bis 31, dadurch gekennzeichnet, daß man das in Wasser oder physiologischer Salzlösung gelöste oder suspendierte paramagnetische Komplexsalz, gegebenenfalls mit den in der Galenik üblichen Zusätzen, in eine für die orale oder intravasale Applikation geeignete Form bringt.

34. Verwendung von mindestens einem physiologisch verträglichen paramagnetischen Komplexsalz aus Aminopolycarbonsäuren der Formeln I bis IV

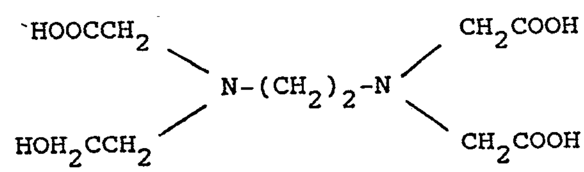

(I)

N-Hydroxyäthyl-N,N',N'-äthylendiamin-triessigsäure (HEDTA),

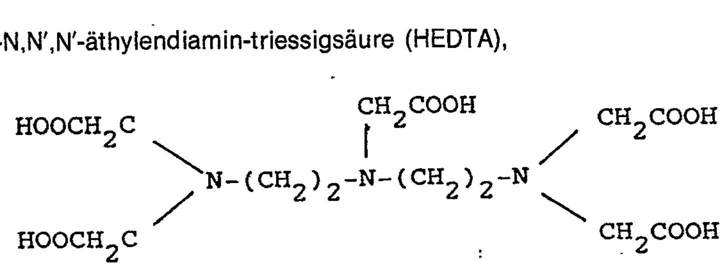

(II)

N,N,N',N",N"-Diäthylentriamin-pentaessigsäure (DTPA),

$$HOH_2C—CH_2N(CH_2COOH)_2 \qquad (III)$$

N-Hydroxyäthyliminodiessigsäure,

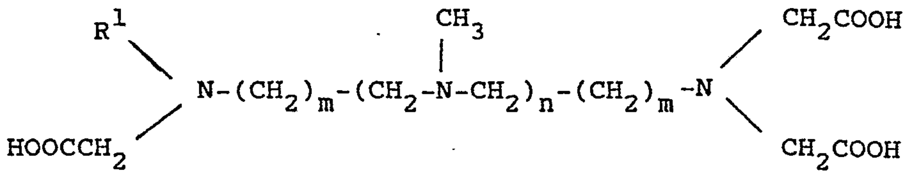

(IV)

worin

m die Zahlen 1 bis 4,

n die Zahlen 0 bis 2,

$R^1$ einen gesättigten oder ungesättigten Kohlenwasserstoffrest mit 4 bis 12 Kohlenwasserstoffatomen oder die Gruppe $—CH_2—COOH$ darstellt,

oder Diphosphonsäuren der allgemeinen Formel V

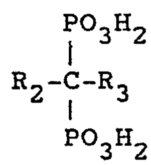

(V)

worin

$R_2$ Wasserstoff, Alkyl mit 1 bis 4 Kohlenstoffatomen, Halogen, die Hydroxy-, Amino- oder $CH_2—COOH$—Gruppe und

$R_3$ Wasserstoff, Alkyl mit 1 bis 4 Kohlenstoffatomen oder die $—CH_2—COOH$—Gruppe bedeuten und den Ionen der Lanthaniden-Elemente der Ordnungszahlen 57 bis 70 oder den Ionen der Übergangsmetalle der Ordnungszahlen 21 bis 29, 42 und 44

und einer organischen Base, wobei als organische Base Glucamin, N-Methylglucamin, N,N-Dimethylglucamin, Äthanolamin, Diäthanolamin, Morpholin, Lysin, Ornithin und Arginin in Betracht kommen, zur Herstellung von Mitteln für die NMR-Diagnostik.

35. Verwendung von mindestens einem physiologisch verträglichen paramagnetischen Komplexsalz nach Anspruch 34, wobei als Aminopolycarbonsäure N,N,N',N'-Äthylendiamin-tetraessigsäure (EDTA) verwendet wird, zur Herstellung von Mitteln für die NMR-Diagnostik.

36. Verwendung von mindestens einem physiologisch verträglichen paramagnetischen Komplexsalz nach Anspruch 34, wobei als Aminopolycarbonsäure N,N,N',N'',N''-Diäthylentriaminpentaessigsäure (DTPA) verwendet wird, zur Herstellung von Mitteln für die NMR-Diagnostik.

37. Verwendung von mindestens einem physiologisch verträglichen paramagnetischen Komplexsalz nach Anspruch 34, wobei als Diphosphonsäure Äthan-1-hydroxy-1,1-diphosphonsäure, Methan-diphosphonsäure oder Äthan-1-amino-1,1-diphosphonsäure verwendet wird, zur Herstellung von Mitteln für di NMR-Diagnostik.

38. Verwendung von Di-N-methylglucaminsalz des Mangan(II)-Komplexes der Äthylendiamintetraessigsäure, Die-N-methylglucaminsalz des Nickel(II)-Komplexes der Äthylendiamin-tetraessigsäure, Tri-N-methylglucaminsalz des Mangan(II)-Komplexes der Diäthylentriamin-pentaessigsäure, N-Methylglucaminsalz des Gadolinium(III)-Komplexes der Äthylendiamin-tetraessigsäure, N-Methylglucaminsalz des Dysprosium(III)-Komplexes der Äthylendiamin-tetraessigsäure, Di-N-Methylglucaminsalz des Holmium(III)-Komplexes der Diäthylentriamin-pentaessigsäure und N-Methylglucaminsalz des Eisen(II)-Komplexes der Äthan-1-hydroxy-1,1-diphosphonsäure zur Herstellung von Mitteln für die NMR-Diagnostik.

39. Verwendung von Di-N-Methylglucaminsalz des Gadolinium(III)-Komplexes der Diäthylentriaminpentaessigsäure zur Herstellung von Mitteln für die NMR-Diagnostik.

40. Verwendung von Di-äthanolaminsalz des Kobalt(II)-Komplexes der Äthylendiamin-tetraessigsäure, Di-diäthanolaminsalz des Kupfer(II)-Komplexes der Äthylendiamin-tetraessigsäure und Tri-diäthanolaminsalz des Mangan(II)-Komplexes der Diäthylentriamin-pentaessigsäure zur Herstellung von Mitteln für die NMR-Diagnostik.

41. Verwendung von Di-morpholinsalz des Mangan(II)-Komplexes der Äthylendiamin-tetraessigsäure zur Herstellung von Mitteln für die NMR-Diagnostik.

42. Verwendung von Di-Lysinsalz des Gadolinium(III)-Komplexes der Diäthylentriamin-pentaessigsäure zur Herstellung von Mitteln für die NMR-Diagnostik.

**Patentansprüche** (für den Vertragsstaat AT)

1. Verfahren zur Herstellung von Mitteln für die NMR-Diagnostik, enthaltend mindestens ein paramagnetisches, physiologisch verträgliches Komplexsalz aus Aminopolycarbonsäuren der Formeln I bis IV

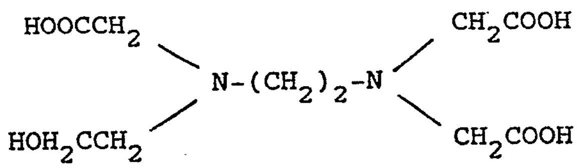

(I)

N-Hydroxyäthyl-N,N',N'-äthylendiamin-triessigsäure (HEDTA),

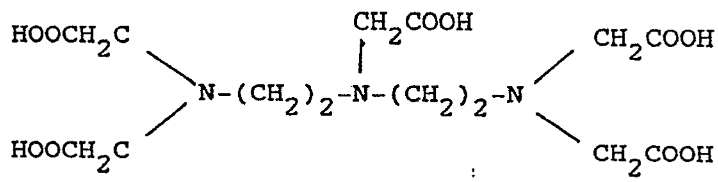

(II)

N,N,N'N'',N''-Diäthylentriamin-pentaessigsäure (DTPA),

$$HOH_2C—CH_2N(CH_2COOH)_2 \qquad (III)$$

N-Hydroxyäthyliminodiessigsäure,

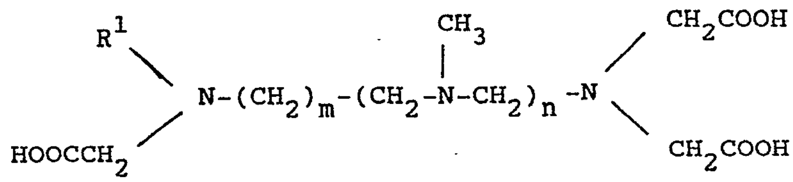

(IV)

worin

m die Zahlen 1 bis 4,

n die Zahlen 0 bis 2,

$R^1$ einen gesättigten oder ungesättigten Kohlenwasserstoffrest mit 4 bis 12 Kohlenwasserstoffatomen oder die Gruppe $—CH_2COOH$ darstellt,

oder Diphosphonsäuren der allgemeinen Formel V

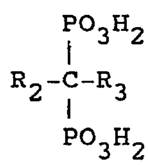

(V)

worin

$R_2$ Wasserstoff, Alkyl mit 1 bis 4 Kohlenstoffatomen, Halogen, die Hydroxy-, Amino- oder $CH_2—COOH—$Gruppe und

$R_3$ Wasserstoff, Alkyl mit 1 bis 4 Kohlenstoffatomen oder die $—CH_2—COOH—$Gruppe bedeuten, und den Ionen der Lanthaniden-Elemente der Ordnungszahlen 57 bis 70 oder den Ionen der Übergangsmetalle der Ordnungszahlen 21 bis 29, 42 und 44

und einer organischen Base, wobei als organische Base Glucamin, N-Methylglucamin, N,N-Dimethylglucamin, Äthanolamin, Diäthanolamin, Morpholin, Lysin, Ornithin und Arginin in Betracht kommen, gegebenenfalls mit den in der Galenik üblichen Zusätzen, gelöst oder suspendiert in Wasser oder physiologischer Salzlösung, dadurch gekennzeichnet, daß man das in Wasser oder physiologischer Salzlösung gelöste oder suspendierte paramagnetische Komplexsalz, gegebenenfalls mit den in der Galenik üblichen Zusätzen, in eine für die orale oder intravasale Applikation geeignete Form bringt.

2. Verfahren nach Anspruch 1, wobei als Aminopolycarbonsäure N,N,N',N'-Äthylendiamintetraessigsäure (EDTA) verwendet wird.

3. Verfahren nach Anspruch 1, wobei als Aminopolycarbonsäure N,N,N',N'',N''-Diäthylentriaminpentaessigsäure (DTPA) verwendet wird.

4. Verfahren nach Anspruch 1, wobei mindestens ein paramagnetisches, physiologisch verträgliches Komplexsalz aus einer Aminopolycarbonsäure der Formel I bis IV und einem Ion der Lanthaniden-Elemente der Ordnungszahlen 57 bis 70 und einer organischen Base verwendet wird.

5. Verfahren nach Anspruch 1, wobei mindestens ein paramagnetisches, physiologisch verträgliches Komplexsalz aus einer Aminopolycarbonsäure der Formel I bis IV und einem Ion der Übergangsmetalle der Ordnungszahlen 21 bis 29, 42 und 44 und einer organischen Base verwendet wird.

6. Verfahren nach Anspruch 1, wobei mindestens ein paramagnetisches, physiologisch verträgliches

Komplexsalz aus einer Diphosphonsäure der allgemeinen Formel V und einem Ion der Lanthaniden-Elemente der Ordnungszahlen 57 bis 70 und einer organischen Base verwendet wird.

7. Verfahren nach Anspruch 1, wobei mindestens ein paramagnetisches, physiologisch verträgliches Komplexsalz aus einer Diphosphonsäure der allgemeinen Formel V und einem Ion der Übergangsmetalle der Ordnungszahlen 21 bis 29, 42 und 44 und einer organischen Base verwendet wird.

8. Verfahren nach Anspruch 1, wobei als Diphosphonsäure Äthan-1-hydroxy-1,1-diphosphonsäure, Methan-diphosphonsäure oder Äthan-1-amino-1,1-diphosphonsäure verwendet wird.

9. Verfahren nach Anspruch 1, wobei Di-N-methylglucaminsalz des Mangan (II)-Komplexes der Äthylendiamin-tetraessigsäure, Di-N-methylglucaminsalz des Nickel (III)-Komplexes der Äthylendiamin-tetraessigsäure, Tri-N-methylglucaminsalz des Mangan (II)-Komplexes der Diäthylentriaminpentaessigsäure, N-Methylglucaminsalz des Gadolinium-(III)-Komplexes der Äthylendiamin-tetraessigsäure, N-Methylglucaminsalz des Dysprosium (III)-Komplexes der Äthylendiamin-tetraessigsäure, Di-N-Methylglucaminsalz des Holmium (III)-Komplexes der Diäthylentriamin-pentaessigsäure oder N-Methylglucaminsalz des Eisen (II)-Komplexes der Äthan-1-hydroxy-1,1-diphosphonsäure verwendet wird.

10. Verfahren nach Anspruch 1, wobei Di-N-Methylglucaminsalz des Gadolinium (III)-Komplexes der Diäthylentriamin-pentaessigsäure verwendet wird.

11. Verfahren nach Anspruch 1, wobei Di-äthanolaminsalz des Kobalt (II)-Komplexes der Äthylendiamin-tetraessigsäure, Di-diäthanolaminsalz des Kupfer (II)-Komplexes der Äthylendiamin-tetraessigsäure oder Tri-diäthanolaminsalz des Mangan (II)-Komplexes der Diäthylentriamin-pentaessigsäure verwendet wird.

12. Verfahren nach Anspruch 1, wobei Di-morpholinsalz des Mangan (II)-Komplexes der Äthylendiamintetraessigsäure verwendet wird.

13. Verfahren nach Anspruch 1, wobei Di-Lysinsalz des Gadolinium (III)-Komplexes der Diäthylentriamin-pentaessigsäure verwendet wird.

**Claims** (for the Contracting States : BE, CH, DE, FR, GB, IT, LI, LU, NL, SE)

1. Physiologically compatible paramagnetic complex salts of aminopolycarboxylic acids of the formulae I to IV

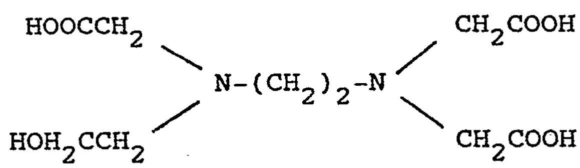

(I)

N-Hydroxyethyl-N,N',N'-ethylenediaminetriacetic acid (HEDTA)

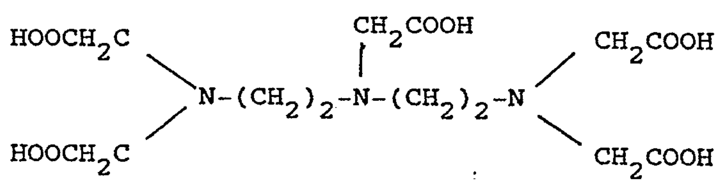

(II)

N,N,N',N'',N''-Diethylenetriaminepentaacetic acid (DTPA)

$$HOH_2C—CH_2N(CH_2COOH)_2 \quad (III)$$

N-Hydroxyethyliminodiacetic acid

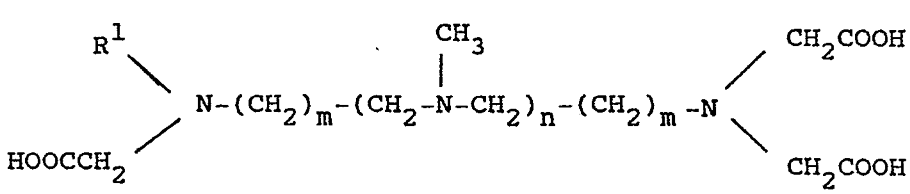

(IV)

wherein

m represents 1 to 4

n represents 0 to 2

$R^1$ represents a saturated or unsaturated hydrocarbon group with 4 to 12 hydrocarbon atoms or the group $—CH_2—COOH$.

or diphosphonic acids of the general formula V

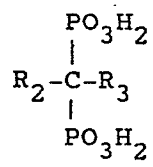 (V)

wherein

$R_2$ represents hydrogen, alkyl of 1 to 4 carbon atoms, halogen, the hydroxy-, amino- or $CH_2$—COOH groups, and

$R_3$ represents hydrogen, alkyl of 1 to 4 carbon atoms, or the —$CH_2$—COOH group,

and the ions of the lanthanide elements of numbers 57 to 70 or the ions of the transition metals of numbers 21 to 29, 42 and 44 and an organic base, by which as organic base glucamine, N-methylglucamine, N,N-dimethylglucamine, ethanolamine, diethanolamine, morpholine, lysine, ornithine and arginine are concerned.

2. Physiologically compatible paramagnetic complex salts according to claim 1 wherein as aminopolycarboxylic acid N,N,N',N'-ethylenediaminetetraacetic acid (EDTA) is used.

3. Physiologically compatible paramagnetic complex salts according to claim 1 wherein as aminopolycarboxylic acid N,N,N',N",N"-diethylenetriaminepentaacetic acid (DTPA) is used.

4. Physiologically compatible paramagnetic complex salts according to claim 1 wherein as diphosphonic acid, ethane-1-hydroxy-1,1-diphosphonic acid, methanediphosphonic acid or ethane-1-amino-1,1-diphosphonic acid is used.

5. Di-N-methylglucamine salt of the manganese(II)-complex of ethylenediaminetetraacetic acid, di-N-methylglucamine salt of the nickel(II)-complex of ethylenediaminetetraacetic acid, tri-N-methylglucamine salt of the manganese(II)-complex of diethylenetriaminepentaacetic acid, N-methylglucamine salt of the gadolinium(III)-complex of ethylenediaminetetraacetic acid, N-methylglucamine salt of the dysprosium(III)-complex of ethylenediaminetetraacetic acid, di-N-methylglucamine salt of the holmium(III)-complex of diethylenetriaminepentaacetic acid and N-methylglucamine salt of the iron(II)-complex of ethane-1-hydroxy-1,1-diphosphonic acid.

6. Di-N-methylglucamine salt of the gadolinium(III)-complex of diethylenetriaminepentaacetic acid.

7. Di-ethanolamine salt of the cobalt(II)-complex of ethylenediaminetetraacetic acid, di-diethanolamine salt of the copper(II)-complex of ethylenediaminetetraacetic acid and tri-diethanolamine salt of the manganese(II)-complex of diethylenetriaminepentaacetic acid.

8. Di-morpholine salt of the manganese(II)-complex of ethylenediaminetetraacetic acid.

9. Di-lysine salt of the gadolinium(III)-complex of diethylenetriaminepentaacetic acid.

10. Physiologically compatible paramagnetic complex salts of aminopolycarboxylic acids of the formulae I to IV

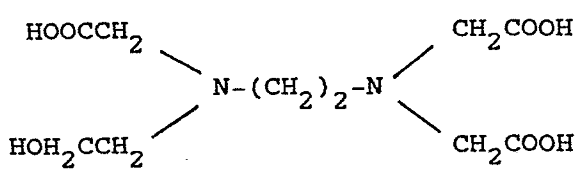 (I)

N-Hydroxyethyl-N,N',N'-ethylenediaminetriacetic acid (HEDTA)

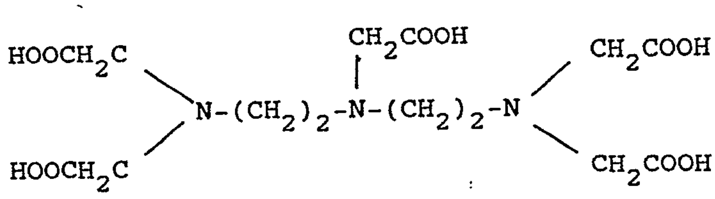 (II)

N,N,N',N",N"-Diethylenetriaminepentaacetic acid (DTPA)

$$HOH_2C—CH_2N(CH_2COOH)_2 \quad (III)$$

N-Hydroxyethyliminodiacetic acid

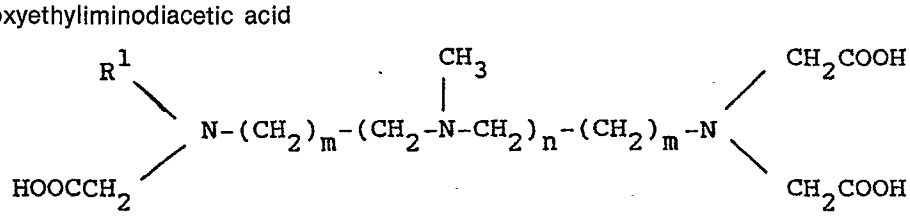 (IV)

wherein

m represents 1 to 4

n represents 0 to 2

$R^1$ represents a saturated or unsaturated hydrocarbon group with 4 to 12 hydrocarbon atoms or the group $—CH_2—COOH$.

or diphosphonic acids of the general formula V

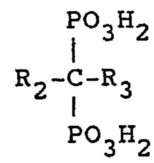

(V)

wherein

$R_2$ represents hydrogen, alkyl of 1 to 4 carbon atoms, halogen, the hydroxy-, amino- or $CH_2—COOH$ groups, and

$R_3$ represents hydrogen, alkyl of 1 to 4 carbon atoms, or the $—CH_2—COOH$ group,

and the ions of the lanthanide elements of numbers 57 to 70 or the ions of the transition metals of numbers 21 to 29, 42 and 44

and an organic base, by which as organic base glucamine, N-methylglucamine, N,N-dimethylglucamine, ethanolamine, diethanolamine, morpholine, lysine, ornithine and arginine are concerned for use in NMR diagnosis.

11. Physiologically compatible paramagnetic complex salts according to claim 10 wherein as aminopolycarboxylic acid N,N,N',N'-ethylenediaminetetraacetic acid (EDTA) is used, for use in NMR diagnosis.

12. Physiologically compatible paramagnetic complex salts according to claim 10 wherein as aminopolycarboxylic acid N,N,N',N",N"-diethylenetriaminepentaacetic acid (DTPA) is used, for use in NMR diagnosis.

13. Physiologically compatible paramagnetic complex salts according to claim 10 wherein as diphosphonic acid, ethane-1-hydroxy-1,1-diphosphonic acid, methane-diphosphonic acid or ethane-1-amino-1,1-diphosphonic acid is used, for use in NMR diagnosis.

14. Di-N-methylglucamine salt of the manganese(II)-complex of ethylenediaminetetraacetic acid, di-N-methylglucamine salt of the nickel(II)-complex of ethylenediaminetetraacetic acid, tri-N-methylglucamine salt of the manganese(II)-complex of diethylenetriaminepentaacetic acid, N-methylglucamine salt of the gadolinium(III)-complex of ethylenediaminetetraacetic acid, N-methylglucamine salt of the dysprosium(III)-complex of ethylenediaminetetraacetic acid, di-N-methylglucamine salt of the holmium(III)-complex of diethylenetriaminepentaacetic acid and N-methylglucamine salt of the iron(II)-complex of ethane-1-hydroxy-1,1-diphosphonic acid for use in NMR diagnosis.

15. Di-N-methylglucamine salt of the gadolinium(III)-complex of diethylenetriaminepentaacetic acid for use in NMR diagnosis.

16. Di-ethanolamine salt of the cobalt(II)-complex of ethylenediaminetetraacetic acid, di-diethanolamine salt of the copper(II)-complex of ethylenediaminetetraacetic acid and tri-diethanolamine salt of the manganese(II)-complex of diethylenetriaminepentaacetic acid for use in NMR diagnosis.

17. Di-morpholine salt of the manganese(II)-complex of ethylenediaminetetraacetic acid for use in NMR diagnosis.

18. Di-lysine salt of the gadolinium(III)-complex of diethylenetriaminepentaacetic acid for use in NMR diagnosis.

19. Material of NMR diagnosis containing at least a paramagnetic, physiologically compatible complex salt of aminopolycarboxylic acids of the formulae I to IV

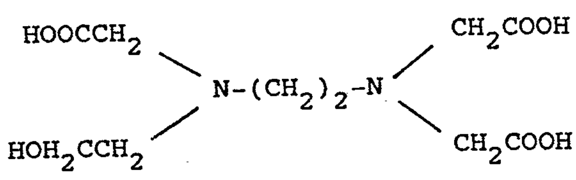

(I)

N-Hydroxyethyl-N,N',N'-ethylenediaminetriacetic acid (HEDTA)

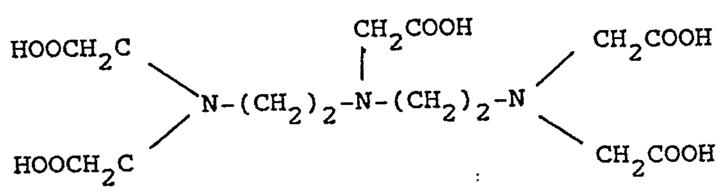

(II)

N,N,N',N'',N''-Diethylenetriaminepentaacetic acid (DTPA)

$$HOH_2C—CH_2N(CH_2COOH)_2 \quad (III)$$

N-Hydroxyethyliminodiacetic acid

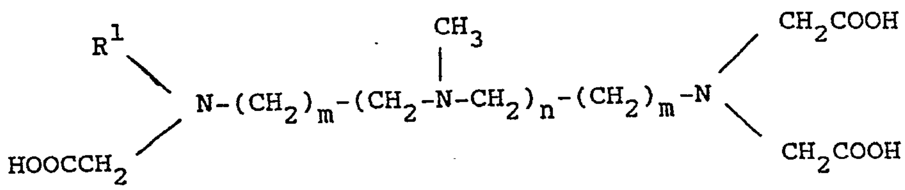

(IV)

wherein
m represents 1 to 4
n represents 0 to 2
$R^1$ represents a saturated or unsaturated hydrocarbon group with 4 to 12 hydrocarbon atoms or the group $—CH_2—COOH$,
or diphosphonic acids of the general formula V

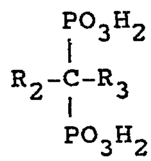

(V)

wherein
$R_2$ represents hydrogen, alkyl of 1 to 4 carbon atoms, halogen, the hydroxy-, amino- or $CH_2—COOH$ groups, and
$R_3$ represents hydrogen, alkyl of 1 to 4 carbon atoms, or the $—CH_2—COOH$ group,
and the ions of the lanthanide elements of numbers 57 to 70 or the ions of the transition metals of numbers 21 to 29, 42 and 44
and an organic base, by which as organic base glucamine, N-methylglucamine, N,N-dimethylglucamine, ethanolamine, diethanolamine, morpholine, lysine, ornithine and arginine are concerned, optionally with the usual additives in the art, dissolved or suspended in water or physiological salt solution.

20. Material for NMR diagnosis according to claim 19 wherein as aminopolycarboxylic acid N,N,N',N'-ethylenediaminetetraacetic acid (EDTA) is used.

21. Material for NMR diagnosis according to claim 19 wherein as aminopolycarboxylic acid N,N,N',N'',N''-diethylenetriaminepentaacetic acid (DTPA) is used.

22. Material for NMR diagnosis according to claim 19, containing at least a paramagnetic, physiologically compatible complex salt of an aminopolycarboxylic acid of formula I to IV and an ion of the lanthanide elements of number 57 to 70 and of an organic base.

23. Material of NMR diagnosis according to claim 19, containing at least a paramagnetic, physiologically compatible salt of an aminopolycarboxylic acid of formula I to IV and an ion of the transition metals of number 21 to 29, 42 and 44 and of an organic base.

24. Material for NMR diagnosis according to claim 19, containing at least a paramagnetic, physiologically compatible complex salt of a diphosphonic acid of the general formula V and an ion of the lanthanide elements of number 57 to 70 and an organic base.

25. Material for NMR diagnosis according to claim 19, containing at least a paramagnetic, physiologically compatible complex salt of a diphosphonic acid of the general formula V and an ion of a transition metal of number 21 to 29, 42 and 44 and an organic base.

26. Material for NMR diagnosis according to claim 19, containing at least a paramagnetic, physiologically compatible complex salt, wherein as diphosphonic acid ethane-1-hydroxy-1,1-diphosphonic acid, methane diphosphonic acid or ethane-1-amino-1,1-diphosphonic acid is used.

27. Material for NMR diagnosis according to claim 19, containing di-N-methylglucamine salt of the manganese(II)-complex of ethylenediaminetetraacetic acid, di-N-methylglucamine salt of the nickel(II)-complex of ethylenediaminetetraacetic acid, tri-N-methylglucamine salt of the manganese(II)-complex of diethylenetriaminepentaacetic acid, N-methylglucamine salt of the gadolinium(III)-complex of ethylenediaminetetraacetic acid, N-methylglucamine salt of the dysprosium(III)-complex of ethylenediaminetetraacetic acid, di-N-methylglucamine salt of the holmium(III)-complex of diethylenetriaminepentaacetic acid and N-methylglucamine salt of the iron(II)-complex of ethane-1-hydroxy-1,1-diphosphonic acid.

28. Material for NMR diagnosis according to claim 19, containing di-N-methylglucamine salt of the gadolinium(III)-complex of diethylenetriaminepentaacetic acid.

29. Material for NMR diagnosis according to claim 19, containing di-ethanolamine salt of the cobalt(II)-complex of ethylenediaminetetraacetic acid, di-diethanolamine salt of the copper(II)-complex of ethylenediaminetetraacetic acid and tri-diethanolamine salt of the manganese(II)-complex of diethylene triaminepentaacetic acid.

30. Material for NMR diagnosis according to claim 19, containing di-morpholine salt of the manganese(II)-complex of ethylenediaminetetraacetic acid.

31. Material for NMR diagnosis according to claim 19, containing di-lysine salt of the gadolinium(III)-complex of diethylenetriaminepentaacetic acid.

32. Material for NMR diagnosis according to claims 19 to 31 characterised in that it contains 5 to 250 mMol/l complex salt.

33. Process for the preparation of the material according to claims 19 to 31, characterised in that one brings into a form for oral or intravascular application the paramagnetic complex salt dissolved or suspended in water or a physiological salt solution optionally with the usual additives in the art.

34. Use of at least one physiologically compatible paramagnetic complex salt or aminopolycarboxylic acids of the formulae I to IV

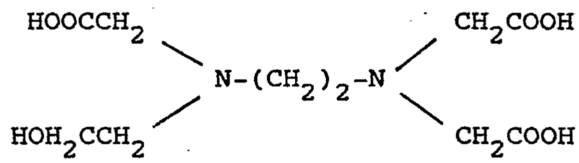

(I)

N-Hydroxyethyl-N,N',N'-ethylenediaminetriacetic acid (HEDTA)

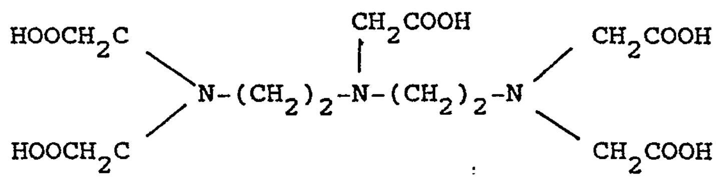

(II)

N,N,N',N'',N''-Diethylenetriaminepentaacetic acid (DTPA)

$$HOH_2C—CH_2N(CH_2COOH)_2 \qquad (III)$$

N-Hydroxyethyliminodiacetic acid

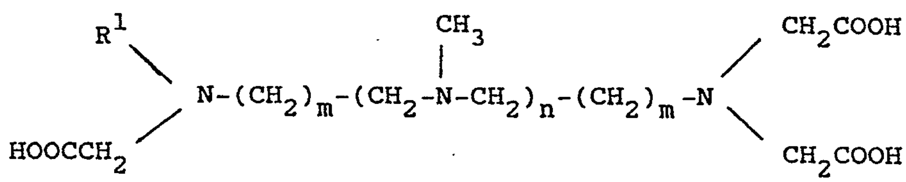

(IV)

wherein

m represents 1 to 4

n represents 0 to 2

$R^1$ represents a saturated or unsaturated hydrocarbon group with 4 to 12 hydrocarbon atoms or the group $—CH_2—COOH$.

or diphosphonic acids of the general formula V

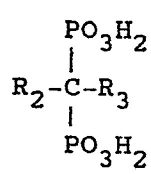

(V)

wherein

$R_2$ represents hydrogen, alkyl of 1 to 4 carbon atoms, halogen, the hydroxy-, amino- or $CH_2—COOH$ groups, and

$R_3$ represents hydrogen, alkyl of 1 to 4 carbon atoms, or the $—CH_2—COOH$ groups,

and the ions of the lanthanide elements of numbers 57 to 70 or the ions of the transition metals of numbers 21 to 29, 42 and 44

and an organic base, by which as organic base glucamine, N-methylglucamine, N,N-dimethylglucamine, ethanolamine, diethanolamine, morphine, lysine, ornithine and arginine are concerned in the preparation of materials for NMR diagnosis.

35. Use of at least one physiologically compatible paramagnetic complex salt according to claim 34, wherein as aminopolycarboxylic acid N,N,N',N'-ethylenediaminetetraacetic acid is used in the preparation of materials for NMR diagnosis.

36. Use of at least one physiologically compatible paramagnetic complex salt according to claim 34, wherein as aminopolycarboxylic acid N,N,N',N'',N''-diethylenetriaminepentaacetic acid (DTPA) is used in the preparation of materials for NMR diagnosis.

37. Use of at least one physiologically compatible paramagnetic complex salt according to claim 34, wherein as diphosphonic acid, ethane-1-hydroxy-1,1-disphosphonic acid, methane-disphosphonic acid or ethane-1-hydroxy-1,1-diphosphonic acid, methane-disphosphonic acid or ethane-1-amino-1,1-diphosphonic acid is used in the preparation of materials for NMR diagnosis.

38. Use of di-N-methylglucamine salt of the manganese(II)-complex of ethylenediaminetetraacetic acid, di-N-methylglucamine salt of the nickel(II)-complex of ethylenediaminetetraacetic acid, tri-N-methylglucamine salt of the manganese(II)-complex of diethylenetriaminepentaacetic acid, N-methylglucamine salt of the gadolinium(III)-complex of ethylene-diaminetetraacetic acid, N-methylglucamine salt of the dysprosium(III)-complex of ethylenediaminetetraacetic acid, di-N-methylglucamine salt of the holmium(III)-complex of diethylenetriaminepentaacetic acid and N-methylglucamine salt of the iron(II)-complex of ethane-1-hydroxy-1,1-diphosphonic acid in the preparation of materials for NMR diagnosis.

39. Use of di-N-methylglucamine salt of the gadolinium(III)-complex of diethylenetriaminepentaacetic acid.

40. Use of di-ethanolamine salt of the cobalt(II)-complex of ethylenediaminetetraacetic acid, di-diethanolamine salt of the copper(II)-complex of ethylenediaminetetraacetic acid and tri-diethanolamine salt of the manganese(II)-complex of diethylenetriaminepentaacetic acid in the preparation of materials for NMR diagnosis.

41. Use of di-morpholine salt of the manganese(II)-complex of ethylenediaminetetraacetic acid in the preparation of materials for NMR diagnosis.

42. Use of di-lysine salt of the gadolinium(III)-complex of diethylenetriaminepentaacetic acid in the preparation of materials for NMR diagnosis.

**Claims** (for the Contracting State AT)

1. Process for the preparation of materials for NMR-diagnosis containing at least a paramagnetic, physiologically compatible complex salt of aminopolycarboxylic acids of the formulae I to IV

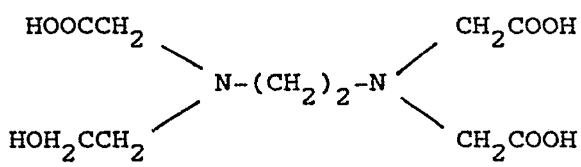

(I)

N-Hydroxyethyl-N,N',N'-ethylenediaminetriacetic acid (HEDTA)

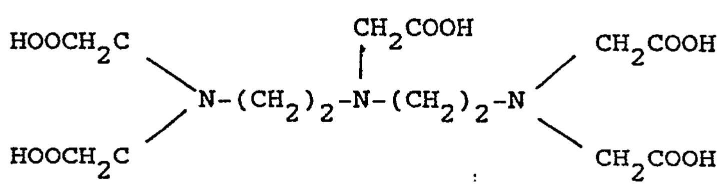

(II)

N,N,N',N'',N''-Diethylenetriaminepentaacetic acid (DTPA)

$$HOH_2C—CH_2N(CH_2COOH)_2 \quad (III)$$

N-Hydroxyethyliminodiacetic acid

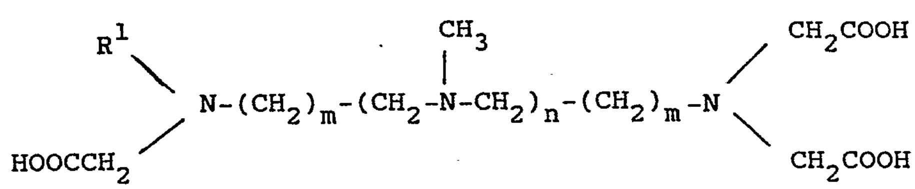

(IV)

wherein

m represents 1 to 4

n represents 0 to 2

$R^1$ represents a saturated or unsaturated hydrocarbon group with 4 to 12 hydrocarbon atoms or the group —$CH_2$—COOH,
or diphosphonic acids of the general formula V

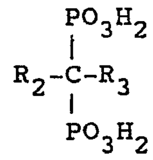

(V)

wherein

$R_2$ represents hydrogen, alkyl of 1 to 4 carbon atoms, halogen, the hydroxy-, amino- or $CH_2$—COOH groups, and

$R_3$ represents hydrogen, alkyl of 1 to 4 carbon atoms, or the —$CH_2$—COOH group,
and the ions of the lanthanide elements of numbers 57 to 70 or the ions of the transition metals of numbers 21 to 29, 42 and 44
and an organic base, by which as organic base glucamine, N-methylglucamine, N,N-dimethylglucamine, ethanolamine, diethanolamine, morpholine, lysine, ornithine and arginine are concerned, optionally with the usual additives in the art, dissolved or suspended in water or physiological salt solution characterised in that one brings into a form for oral or intravascular application the paramagnetic complex salt dissolved or suspended in water or a physiological salt solution optionally with the usual additives in the art.

2. Process according to claim 1 wherein as aminopolycarboxylic acid N,N,N',N'-ethylenediaminetetraacetic acid (ETDA) is used.

3. Process according to claim 1 wherein as aminopolycarboxylic acid N,N,N',N",N"-diethylenetriaminepentaacetic acid (DTPA) is used.

4. Process according to claim wherein at least a paramagnetic, physiologically compatible complex salt of an aminopolycarboxylic acid of formula I to IV and an ion of the lanthanide elements of number 57 to 70 and of an organic base is used.

5. Process according to claim 1 wherein at least a paramagnetic, physiologically compatible salt of an aminopolycarboxylic acid of formula I to IV and an ion of the transition metals of number 21 to 29, 42 and 44 and of an organic base is used.

6. Process according to claim 1 wherein at least a paramagnetic, physiologically compatible complex salt of a diphosphonic acid of the general formula V and an ion of the lanthanide elements of number 57 to 70 and an organic base is used.

7. Process according to claim 1 wherein at least a paramagnetic, physiologically compatible complex salt of a diphosphonic acid of the general formula V and an ion of a transition metal of number 21 to 29, 42 and 44 and an organic base is used.

8. Process according to claim 1 wherein as diphosphonic acid ethane-1-hydroxy-1,1-diphosphonic acid, methane diphosphonic acid or ethane-1-amino-1,1-diphosphonic acid is used.

9. Process according to claim 1 wherein di-N-methylglucamine salt of the manganese(II)-complex of ethylenediaminetetraacetic acid, di-N-methylglucamine salt of the nickel(II)-complex of ethylenediaminetetraacetic acid, tri-N-methylglucamine salt of the manganese(II)-complex of diethylenetriaminepentaacetic acid, N-methylglucamine salt of the gadolinium(III)-complex of ethylenediaminetetraacetic acid, N-methylglucamine salt of the dysprosium(III)-complex of ethylenediaminetetraacetic acid, di-N-methylglucamine salt of the holmium(III)-complex of diethylenetriaminepentaacetic acid or N-methylglucamine salt of the iron(II)-complex of ethane-1-hydroxy-1,1-diphosphonic acid is used.

10. Process according to claim 1 wherein di-N-methylglucamine salt of the gadolinium(III)-complex of diethylenetriaminepentaacetic acid is used.

11. Process according to claim 1 wherein di-ethanolamine salt of the cobalt(II)-complex of ethylenediaminetetraacetic acid, di-diethanolamine salt of the copper(II)-complex of ethylenediaminetetraacetic acid or tri-diethanolamine salt of the manganese(II)-complex of diethylene triaminepentaacetic acid is used.

12. Process according to claim 1 wherein di-morpholine salt of the manganese(II)-complex of ethylenediaminetetraacetic acid is used.

13. Process according to claim 1 wherein di-lysine salt of the gadolinium(III)-complex of diethylenetriaminepentaacetic acid is used.

**Revendications** (pour les Etats contractants : BE, CH, DE, FR, GB, IT, LI, LU, NL, SE)

1. Sels complexes paramagnétiques physiologiquement tolérés, formés d'acides aminopolycarboxyliques de formules I à IV ci-après :

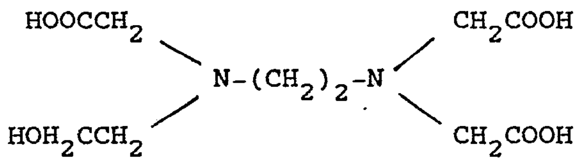

(I)

Acide N-hydroxyéthyl-N,N',N'-éthylène-diamine-triacétique (HEDTA).

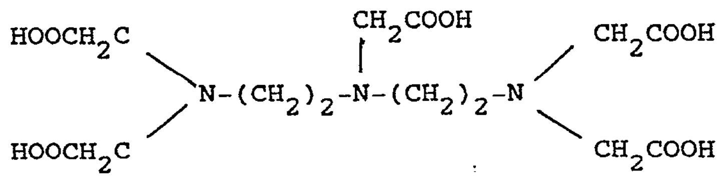

(II)

Acide N,N,N',N'',N''-diéthylène-triamine-pentacétique (DTPA)

$$HOH_2C—CH_2N(CH_2COOH)_2 \quad (III)$$

Acide N-hydroxyéthyl-iminodiacétique

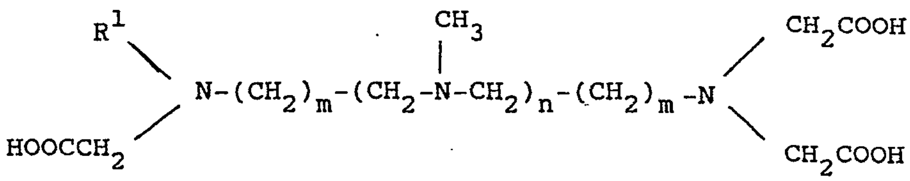

(IV)

m étant un nombre de 1 à 4,
n un nombre de 0 à 2 et
$R^1$ un radical hydrocarboné saturé ou insaturé en $C_4$ à $C_{12}$ ou le groupe $—CH_2—COOH$,
ou bien d'acides disphosphoniques de formule générale V

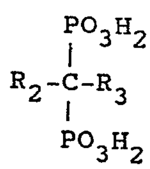

(V)

dans laquelle
$R^2$ représente l'hydrogène, un alkyle en $C_1$ à $C_4$, un halogène ou le groupe hydroxy, amino ou $—CH_2—COOH$ et
$R^3$ l'hydrogène, un alkyle en $C_1$ à $C_4$ ou le groupe $—CH_2—COOH$,
et d'ions de lanthanides des numéros atomiques 57 à 70 ou d'ions de métaux de transition des numéros atomiques 21 à 29, 42 et 44,
avec une base organique pouvant être la glucamine, la N-méthylglucamine, la N,N-diméthylglucamine, l'éthanolamine, la diéthanolamine, la morpholine, la lysine, l'ornithine ou l'arginine.

2. Sels complexes paramagnétiques selon la revendication 1 dans lesquels l'acide aminopolycarboxylique est l'acide N,N,N',N'-éthylène-diamine-tétraacétique (EDTA).

3. Sels complexes paramagnétiques selon la revendication 1 dans lesquels l'acide aminopolycarboxylique est l'acide N,N,N',N'',N''-diéthylène-triamine-pentacétique (DTPA).

4. Sels complexes paramagnétiques selon la revendication 1 dans lesquels l'acide diphosphonique est l'acide éthane-1-hydroxy-1,1-diphosphonique, l'acide méthane-diphosphonique ou l'acide éthane-1-amino-1,1-diphosphonique.

5. Le sel de di-N-méthylglucamine du complexe de manganèse divalent de l'acide éthylène-diamine-tétracétique, le sel de di-N-méthylglucamine du complexe de nickel divalent de l'acide éthylène-diamine-tétracétique, le sel de tri-N-méthylglucamine du complexe de manganèse divalent de l'acide diéthylène-triamine-pentacétique, le sel de N-méthylglucamine du complexe de gadolinium trivalent de l'acide éthylène-diamine-tétracétique, le sel de N-méthylglucamine du complexe de dysprosium trivalent de l'acide éthylène-diamine-tétracétique, le sel de di-N-méthylglucamine du complexe d'holmium trivalent de l'acide diéthylène-triamine-pentacétique et le sel de N-méthylglucamine du complexe de fer divalent de l'acide éthane-1-hydroxy-1,1-diphosphonique.

6. Le sel de di-N-méthylglucamine du complexe de gadolinium trivalent de l'acide diéthylène-triamine-pentacétique.

7. Le sel de diéthanolamine du complexe de cobalt divalent de l'acide éthylène-diamine-tétracétique,

le sel de di-diéthanolamine du complexe de cuivre divalent de l'acide éthylène-diamine-tétracétique et le sel de tridiéthanolamine du complexe de manganèse divalent de l'acide diéthylène-triamine-pentacétique.

8. Le sel de dimorpholine du complexe de manganèse divalent de l'acide éthylène-diamine-tétracétique.

9. Le sel de di-lysine du complexe de gadolinium trivalent de l'acide diéthylène-triamine-pentacétique.

10. Sels complexes paramagnétiques physiologiquement tolérés, formés d'acides aminopolycarboxyliques de formules I à IV ci-après :

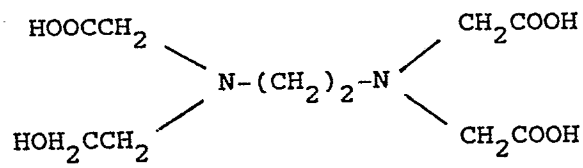

(I)

Acide N-hydroxyéthyl-N,N',N'-éthylène-diamine-triacétique (HEDTA).

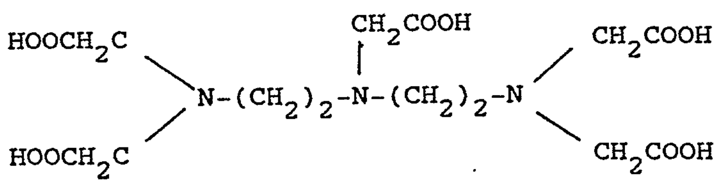

(II)

Acide N,N,N',N'',N''-diéthylène-triamine pentacétique (DTPA).

$$HOH_2C—CH_2N(CH_2COOH)_2 \quad (III)$$

Acide N-hydroxyéthyl-iminodiacétique

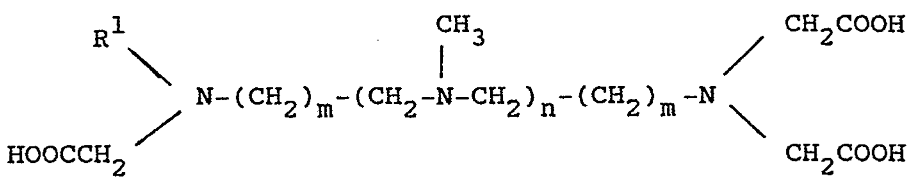

(IV)

m étant un nombre de 1 à 4,
n un nombre de 0 à 2 et
$R^1$ un radical hydrocarboné saturé ou insaturé en $C_4$ à $C_{12}$ ou le groupe $—CH_2—COOH—$,
ou bien d'acides diphosphoniques de formule générale V

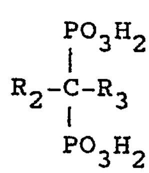

(V)

dans laquelle
$R^2$ représente l'hydrogène, un alkyle en $C_1$ à $C_4$, un halogène ou le groupe hydroxy, amino ou $—CH_2—COOH$ et
$R^3$ l'hydrogène, un alkyle en $C_1$ à $C_4$ ou le groupe $—CH_2—COOH$,
et d'ions de lanthanides des numéros atomiques 57 à 70 ou d'ions de métaux de transition des numéros atomiques 21 à 29, 42 et 44,
avec une base organique pouvant être la glucamine, la N-méthylglucamine, la N-N-diméthylglucamine, l'éthanolamine, la diéthanolamine, la morpholine, la lysine, l'ornithine ou l'arginine, pour leur emploi dans le diagnostic par RMN.

11. Sels complexes paramagnétiques selon la revendication 10 dans lesquels l'acide aminopolycarboxylique est l'acide N,N,N'-N'-éthylène-diamine-tétracétique (EDTA), pour leur emploi dans le diagnostic par RMN.

12. Sels complexes paramagnétiques selon la revendication 10, dans lesquels l'acide aminopolycarboxylique est l'acide N,N,N',N'',N''-diéthylène-triamine-pentacétique (DTPA), pour leur emploi dans le diagnostic par RMN.

13. Sels complexes paramagnétiques selon la revendication 10 dans lesquels l'acide diphosphonique est l'acide éthane-1-hydroxy-1,1-diphosphonique, l'acide méthane-diphosphonique ou l'acide éthane-1-amino-1,1-diphosphonique pour leur emploi dans le diagnostic par RMN.

14. Le sel de di-N-méthylglucamine du complexe de manganèse divalent de l'acide éthylène-diamine-tétracétique, le sel de di-N-méthylglucamine du complexe de nickel divalent de l'acide éthylène-diamine-tétracétique, le sel de tri-N-méthylglucamine du complexe de manganèse divalent de l'acide diéthylène-triamine-pentacétique, le sel de N-méthylglucamine du complexe de gadolinium trivalent de l'acide éthylène-diamine-tétracétique, le sel de N-méthylglucamine du complexe de dysprosium trivalent de l'acide éthylène-diamine-tétracétique, le sel de di-N-méthylglucamine du complexe d'holmium trivalent de l'acide diéthylène-triamine-pentacétique et le sel de N-méthylglucamine du complexe de fer divalent de l'acide éthane-1-hydroxy-1,1-diphosphonique, pour leur emploi dans le diagnostic pour RMN.

15. Le sel de di-N-méthylglucamine du complexe de gadolinium trivalent de l'acide diéthylène-triamine-pentacétique pour son emploi dans le diagnostic par RMN.

16. Le sel de diéthanolamine du complexe de cobalt divalent de l'acide éthylène-diamine-tétracétique, le sel de di-diéthanolamine du complexe de cuivre divalent de l'acide éthylène-diamine-tétracétique et le sel de tri-diéthanolamine du complexe de manganèse divalent de l'acide diéthylène-triamine-pentacétique pour leur emploi dans le diagnostic par RMN.

17. Le sel de dimorpholine du complexe de manganèse divalent de l'acide éthylène-diamine-tétracétique pour son emploi dans le diagnostic par RMN.

18. Le sel de di-lysine du complexe de gadolinium trivalent de l'acide diéthylène-triamine-pentacétique pour son emploi dans le diagnostic par RMN.

19. Agents de contraste pour le diagnostic par RMN, contenant un ou plusieurs sels complexes paramagnétiques physiologiquement tolérés, formés d'acides aminopolycarboxyliques de formules I à IV

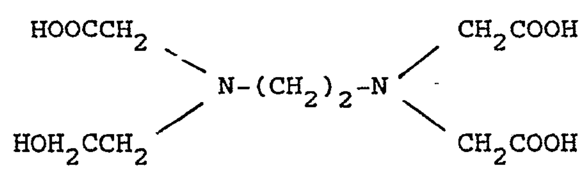 (I)

Acide N-hydroxyéthyl-N,N'-N'-éthylène-diamine-triacétique (HEDTA).

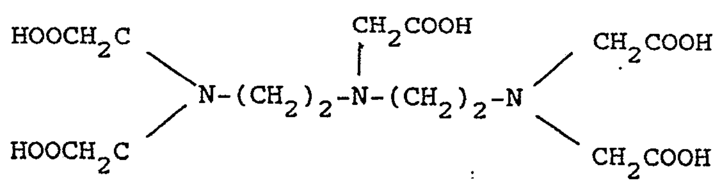 (II)

Acide N,N,N',N'',N''-diéthylène-triamine-pentacétique (DTPA).

$$HOH_2C—CH_2N(CH_2COOH)_2 \quad (III)$$

Acide N-hydroxyéthyl-iminodiacétique

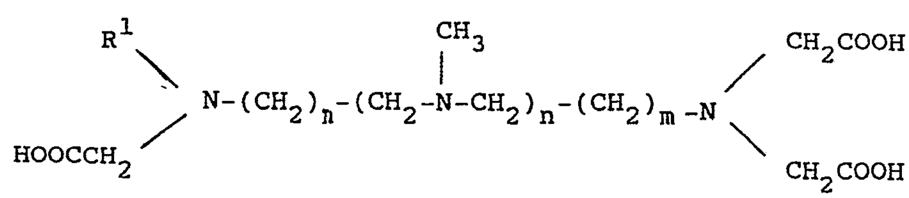 (IV)

m étant un nombre de 1 à 4,

n un nombre de 0 à 2, et

$R_1$ un radical hydrocarboné saturé ou insaturé en $C_4$ à $C_{12}$ ou le groupe $—CH_2COOH$,

ou bien d'acides diphosphoniques de formule générale V

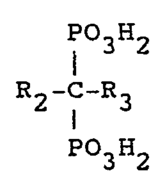 (V)

dans laquelle

$R^2$ représente l'hydrogène, un alkyle en $C_1$ à $C_4$, un halogène ou le groupe hydroxy, amino ou $—CH_2—COOH—$ et

$R^3$ l'hydrogène, un alkyle en $C_1$ à $C_4$ ou le groupe $—CH_2—COOH$,

et d'ions de lanthanides des numéros atomiques 57 à 70 ou d'ions de métaux des transitions de numéros atomiques 21 à 29, 42 et 44,

avec une base organique pouvant être la glucamine, la N-méthylglucamine, la N,N-diméthylglucamine, l'éthanolamine, la diéthanolamine, la morpholine, la lysine, l'ornithine ou l'arginine, éventuellement avec les additifs courants en pharmacie galénique, en solution ou en suspension dans de l'eau ou dans du sérum physiologique.

20. Agents de contraste selon la revendication 19, dans lesquels l'acide aminopolycarboxylique est l'acide N,N,N',N'-éthylène-diamine-tétracétique (EDTA).

21. Agents de contraste selon la revendication 19 dans lesquels l'acide aminopolycarboxylique est l'acide N,N,N',N'',N''-diéthylène-triamine-pentacétique (DTPA).

22. Agents de contraste selon la revendication 19 contenant un ou plusieurs sels complexes paramagnétiques physiologiquement tolérés formés d'un acide aminopolycarboxylique de formule I à IV et d'un ion des lanthanides de numéros atomiques 57 à 70 avec une base organique.

23. Agents de contraste selon la revendication 19 contenant un ou plusieurs sels complexes paramagnétiques formés d'un acide aminopolycarboxylique de formule I à IV et d'un ion des métaux de transition de numéros atomiques 21 à 29, 42 et 44, avec une base organique.

24. Agents de contraste selon la revendication 19 contenant un ou plusieurs sels complexes paramagnétiques formés d'un acide diphosphonique de formule générale V et d'un ion des lanthanides de numéros atomiques 57 à 70, avec une base organique.

25. Agents de contraste selon la revendication 19 contenant un ou plusieurs sels complexes paramagnétiques formés d'un acide diphosphonique de formule générale V et d'un ion des métaux de transition de numéros atomiques 21 à 29, 42 et 44, avec une base organique.

26. Agents de contraste selon la revendication 19 contenant un ou plusieurs sels complexes paramagnétiques formés avec l'acide éthane-1-hydroxy-1,1-diphosphonique, méthane-diphosphonique ou éthane-1-amino-1,1-diphosphonique.

27. Agents de contraste selon la revendication 19 contenant le sel de di-N-méthylglucamine du complexe de manganèse divalent de l'acide éthylène-diamine-tétracétique, le sel de di-N-méthylglucamine du complexe de nickel divalent de l'acide éthylène-diamine-tétracétique, le sel de tri-N-méthylglucamine du complexe de manganèse divalent de l'acide diéthylène-triamine-pentacétique, le sel de N-méthylglucamine du complexe de gadolinium trivalent de l'acide éthylène-diamine-tétracétique, le sel de N-méthylglucamine du complexe de dysprosium trivalent de l'acide éthylène-diamine-tétracétique, le sel de di-N-méthylglucamine du complexe d'holmium trivalent de l'acide diéthylène-triamine-pentacétique ou le sel de N-méthylglucamine du complexe de fer divalent de l'acide éthane-1-hydroxy-1,1-diphosphonique.

28. Agent de contraste selon la revendication 19 contenant le sel de di-N-méthylglucamine du complexe de gadolinium trivalent de l'acide diéthylène-triamine-pentacétique.

29. Agent de contraste selon la revendication 19 contenant le sel de diéthanolamine du complexe de cobalt divalent de l'acide éthylène-diamine-tétracétique, le sel de di-diéthanolamine du complexe de cuivre divalent de l'acide éthylène-diamine-tétracétique ou le sel de tridiéthanolamine du complexe de manganèse divalent de l'acide diéthylène-diamine-pentacétique.

30. Agent de contraste selon la revendication 19 contenant le sel de dimorpholine du complexe de manganèse divalent de l'acide éthylène-diamine-tétracétique.

31. Agent de contraste selon la revendication 19 contenant le sel de di-lysine du complexe de gadolinium trivalent de l'acide diéthylène-triamine-pentacétique.

32. Agents de contraste selon l'une quelconque des revendications 19 à 31, caractérisés en ce qu'ils contiennent de 5 à 250 mmoles/l du sel complexe.

33. Procédé de préparation des agents de contraste selon l'une quelconque des revendications 19 à 31, procédé caractérisé en ce que l'on met sous une forme propre à l'administration orale ou intravasculaire le sel complexe paramagnétique dissous ou en suspension dans de l'eau ou dans du sérum physiologique, éventuellement avec les additifs courants en pharmacie galénique.

34. L'emploi, pour la préparation d'agents de contraste pour le diagnostic par RMN, d'un ou de plusieurs sels complexes paramagnétiques physiologiquement tolérés, formés d'acides aminopolycarboxyliques de formules I à IV ci-après :

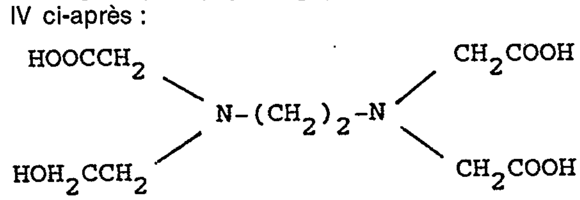

(I)

Acide N-hydroxyéthyl-N,N',N'-éthylène-diamine-triacétique (HEDTA).

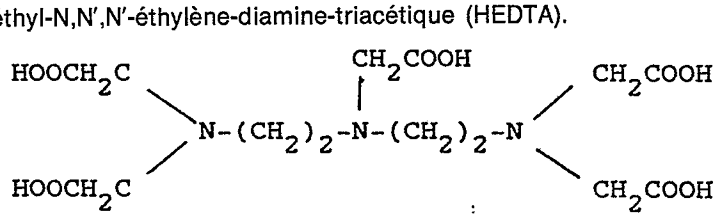

(II)

Acide N,N,N',N",N"-diéthylène-triamine-pentacétique (DTPA).

$$HOH_2C—CH_2N(CH_2COOH)_2 \qquad (III)$$

Acide N-hydroxyéthyl-iminodiacétique

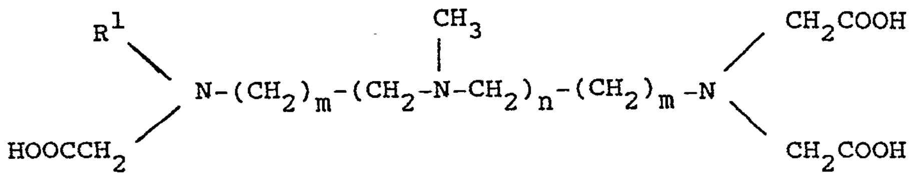

(IV)

m étant un nombre de 1 à 4,
n un nombre de 0 à 2 et
$R^1$ un radical hydrocarboné saturé ou insaturé en $C_4$ à $C_{12}$ ou le groupe $—CH_2—COOH$,
ou bien d'acides diphosphoniques de formule générale V

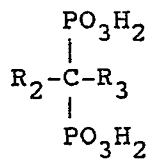

(V)

dans laquelle
$R^2$ représente l'hydrogène, un alkyle en $C_1$ à $C_4$, un halogène ou le groupe hydroxy, amino ou $—CH_2COOH$ et
$R^3$ l'hydrogène, un alkyle en $C_1$ à $C_4$ ou le groupe $—CH_2COOH$,
et d'ions de lanthanides des numéros atomiques 57 à 70 ou d'ions de métaux de transition des numéros atomiques 21 à 29, 42 et 44,
avec une base organique pouvant être la glucamine, la N-méthylglucamine, la N,N-diméthylglucamine, l'éthanolamine, la diéthanolamine, la morpholine, la lysine, l'ornithine, ou l'arginine.

35. L'emploi, pour la préparation d'agents de contraste pour le diagnostic par RMN, d'un ou de plusieurs sels complexes paramagnétiques selon la revendication 34 dans lesquels l'acide aminopolycarboxylique est l'acide N,N,N',N'-éthylène-diamine-tétracétique (EDTA).

36. L'emploi, pour la préparation d'agents de contraste pour le diagnostic par RMN, d'un ou de plusieurs sels complexes paramagnétiques selon la revendication 34 dans lesquels l'acide aminopolycarboxylique est l'acide N,N,N',N",N"-diéthylène-triamine-pentacétique (DTPA).

37. L'emploi, pour la préparation d'agents de contraste pour le diagnostic par RMN, d'un ou de plusieurs sels complexes paramagnétiques selon la revendication 34 dans lesquels l'acide diphosphonique est l'acide éthane-1-hydroxy-1,1-diphosphonique, l'acide méthane-diphosphonique ou l'acide éthane-1-amino-1,1-diphosphonique.

38. L'emploi, pour la préparation d'agents de contraste pour le diagnostic par RMN, du sel de di-N-méthylglucamine du complexe de manganèse divalent de l'acide éthylène-diamine-tétracétique, du sel de di-N-méthylglucamine du complexe de nickel divalent de l'acide éthylène-diamine-tétracétique, du sel de tri-N-méthylglucamine du complexe de manganèse divalent de l'acide diéthylène-triamine-pentacétique, du sel de N-méthyl-glucamine du complexe de gadolinium trivalent de l'acide éthylène-diamine-tétracétique, du sel de N-méthylglucamine du complexe de dysprosium trivalent de l'acide éthylène-diamine-tétracétique, du sel de di-N-méthylglucamine du complexe d'holmium trivalent de l'acide diéthylène-triamine-pentacétique et du sel de N-méthylglucamine du complexe de fer divalent de l'acide éthane-1-hydroxy-1,1-diphosphonique.

39. L'emploi, pour la préparation d'agents de contraste pour le diagnostic par RMN, du sel de di-N-méthylglucamine du complexe de gadolinium trivalent de l'acide diéthylène-triamine-pentacétique.

40. L'emploi, pour la préparation d'agents de contraste pour le diagnostic par RMN, du sel de diéthanolamine du complexe de cobalt divalent de l'acide éthylène-diamine-tétracétique, du sel de di-diéthanolamine du complexe de cuivre divalent de l'acide éthylène-diamine-tétracétique et du sel de tri-diéthanolamine du complexe de manganèse divalent de l'acide diéthylène-triamine-pentacétique.

41. L'emploi, pour la préparation d'agents de contraste pour le diagnostic par RMN, du sel de dimorpholine du complexe de manganèse divalent de l'acide éthylène-diamine-tétracétique.

42. L'emploi, pour la préparation d'agents de contraste pour le diagnostic par RMN, du sel de di-lysine du complexe de gadolinium trivalent de l'acide diéthylène-triamine-pentacétique.

**Revendications** (pour l'Etat Contractant AT)

1. Procédé de préparation d'agents de contraste pour des diagnostics par RMN, contenant un ou

plusieurs sels complexes paramagnétiques physiologiquement tolérés, d'acides amino-polycarboxyliques de formules I à IV ci-après

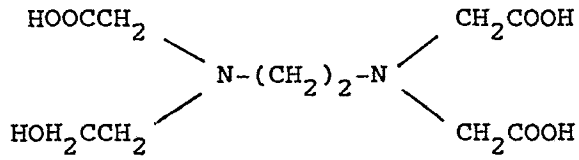

(I)

Acide N-hydroxyéthyl-N,N',N'-éthylène-diamine-triacétique (HEDTA).

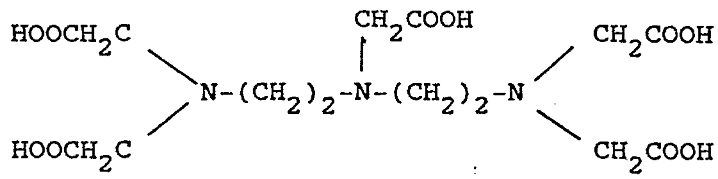

(II)

Acide N,N,N',N'',N''-diéthylène-triamine-pentacétique (DTPA)

$$HOH_2C—CH_2(CH_2COOH)_2 \qquad (III)$$

Acide N-hydroxyéthyl-iminodiacétique

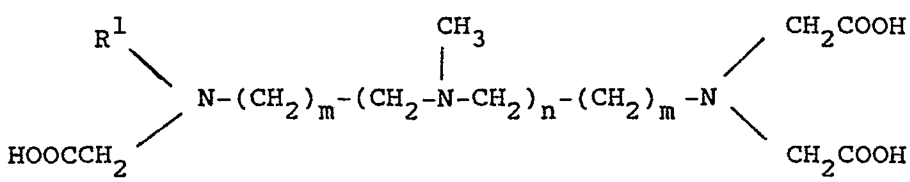

(IV)

m étant un nombre de 1 à 4,

n un nombre de 0 à 2 et

$R^1$ un radical hydrocarboné saturé ou insaturé en $C_4$ à $C_{12}$ ou le groupe $—CH_2—COOH$,

ou bien d'acides disphosphoniques de formule générale V

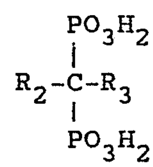

(V)

dans laquelle

$R^2$ représente l'hydrogène, un alkyle en $C_1$ à $C_4$, un halogène ou le groupe hydroxy, amino ou $—CH_2—COOH$ et

$R^3$ l'hydrogène, un alkyle en $C_1$ à $C_4$ ou le groupe $—CH_2—COOH$,

et d'ions de lanthanides des numéros atomiques 57 à 70 ou d'ions de métaux de transition des numéros atomiques 21 à 29, 42 et 44,

avec une base organique pouvant être la glucamine, la N-méthylglucamine, la N,N-diméthylglucamine, l'éthanolamine, la diéthanolamine, la morpholine, la lysine, l'ornithine ou l'arginine, éventuellement avec les additifs courants en pharmacie galénique, en solution ou en suspension dans de l'eau ou dans du sérum physiologique, procédé caractérisé en ce que l'on met sous une forme appropriée à l'administration par la voie orale ou intravasculaire le sel complexe paramagnétique dissous ou mis en suspension dans l'eau ou dans du sérum physiologique, éventuellement avec les additifs courants en galénique.

2. Procédé selon la revendication 1 dans lequel l'acide aminopolycarboxylique est l'acide N,N,N',N'-éthylène-diamine-tétraacétique (EDTA).

3. Procédé selon la revendication 1 dans lequel l'acide aminopolycarboxylique est l'acide N,N,N',N'',N''-diéthylène-triamine-pentaacétique (DTPA).

4. Procédé selon la revendication 1 dans lequel on utilise un ou plusieurs sels complexes paramagnétiques d'un acide aminopolycarboxylique de formule I à IV et d'un ion des lanthanides de numéros atomiques 57 à 70 avec une base organique.

5. Procédé selon la revendication 1 dans lequel on utilise un ou plusieurs sels paramagnétiques d'un acide aminopolycarboxylique de formule I à IV et d'un ion des métaux de transition de numéros atomiques 21 à 29, 42 et 44, avec une base organique.

6. Procédé selon la revendication 1 dans lequel on utilise un ou plusieurs sels complexes

paramagnétiques d'un acide diphosphonique de formule générale V et d'un ion des lanthanides de numéros atomiques 57 à 70 avec une base organique.

7. Procédé selon la revendication 1 dans lequel on utilise un ou plusieurs sels complexes paramagnétiques d'un acide diphosphonique de formule V et d'un ion des métaux de transition de numéros atomiques 21 à 29, 42 et 44, avec une base organique.

8. Procédé selon la revendication 1 dans lequel l'acide diphosphonique est l'acide éthane-1-hydroxy-1,1-diphosphonique, l'acide méthane-diphosphonique ou l'acide éthane-1-amino-1,1-diphosphonique.

9. Procédé selon la revendication 1 dans lequel on utilise le sel de di-N-méthylglucamine du complexe de manganèse divalent de l'acide éthylène-diamine-tétracétique, sel de di-N-méthyl-glucamine du complexe de nickel divalent de l'acide éthylène-diamine-tétracétique, le sel de tri-N-méthyl-glucamine du complexe de manganèse divalent de l'acide diéthylène-triamine-penta-acétique, le sel de N-méthylglucamine du complexe de gadolinium trivalent de l'acide éthylène-diamine-tétracétique, le sel de N-méthylglucamine du complexe de dysprosium trivalent de l'acide éthylène-diamine tétra-acétique, le sel de di-N-méthylglucamine du complexe d'holmium trivalent de l'acide diéthylène-triamine-penta-acétique ou le sel de N-méthyl-glucamine du complexe ferreux de l'acide éthane-1-hydroxy-1,1-diphosphonique.

10. Procédé selon la revendication 1 dans lequel on utilise le sel de di-N-méthyl-glucamine du complexe de gadolinium trivalent de l'acide diéthylène-triamine-pentacétique.

11. Procédé selon la revendication 1 dans lequel on utilise le sel de diéthanolamine du complexe de cobalt divalent de l'acide éthylène-diamine-tétracétique, le sel de di-diéthanolamine du complexe de cuivre divalent de l'acide éthylène-diamine-tétracétique ou le sel de tri-diéthanolamine du complexe de manganèse divalent de l'acide diéthylène-triamine-pentacétique.

12. Procédé selon la revendication 1 dans lequel on utilise le sel de dimorpholine du complexe de manganèse divalent de l'acide éthylène-diamine-tétracétique.

13. Procédé selon la revendication 1 dans lequel on utilise le sel de dilysine du complexe de gadolinium trivalent de l'acide diéthylène-triamine-pentacétique.